# EUROPEAN PATENT SPECIFICATION

(11) **EP 3 975 864 B1**
(45) Date of publication and mention of the grant of the patent: **01.05.2024**
(21) Application number: 20727288.1
(22) Date of filing: 21.05.2020
(51) Int. Cl.: A61B 8/08, A61B 8/00

(54) **ENCODED SYNCHRONIZED MEDICAL INTERVENTION IMAGE SIGNALS AND SENSOR SIGNALS**
CODIERTE SYNCHRONISIERTE MEDIZINISCHE EINGRIFFSBILDSIGNALE UND SENSORSIGNALE
SIGNAUX D'IMAGE D'INTERVENTION MÉDICALE ET SIGNAUX DE CAPTEUR SYNCHRONISÉS CODÉS

(30) Priority: 30.05.2019 US 201962854739 P; 31.07.2019 EP 19189315
(43) Date of publication of application: 06.04.2022
(73) Proprietor: Koninklijke Philips N.V., 5656 AG Eindhoven (NL)
(72) Inventor: ERKAMP, Ramon, Quido, 5656 AE Eindhoven (NL); JAIN, Ameet, Kumar, 5656 AE Eindhoven (NL); CHEN, Alvin, 5656 AE Eindhoven (NL); BHARAT, Shyam, 5656 AE Eindhoven (NL); VAIDYA, Kunal, 5656 AE Eindhoven (NL)
(74) Representative: Philips Intellectual Property & Standards
(86) International application number: PCT/EP2020/064220
(87) International publication number: WO 2020/239610

(56) References cited:
- WO-A1-2018/116114
- WO-A2-2014/016736
- US-A- 6 122 538

## Description

### BACKGROUND OF THE INVENTION

A technology for medical device tracking during ultrasound imaging is currently being developed. In one application the use of this technology in real-time tracking of a needle tip during peripheral nerve block procedures during 2D ultrasound imaging is contemplated. The use of this technology to track other interventional devices during other interventional procedures, such as for example guide wires during a peripheral vascular intervention, during either 2D or 3D ultrasound imaging, is also contemplated. US 6 122 538 discloses a system and method for improved accuracy and reliability in the spatial tracking of medical devices which are to be manipulated in a freehand manner using motion sensors.

For accurate tracking with technology involving passive ultrasound sensors it is important to know the precise timing between a received sensor signal and the timing of associated acoustic transmit events in the ultrasound imaging probe. There are circumstances where latencies in a transmission channel of the sensor signal can be imposed. The encoded synchronized medical intervention image signals and sensor signals described herein provide an efficient method of obtaining the precise timing information between the received sensor signal and the acoustic transmit events in the ultrasound imaging probe to preempt latencies imposed subsequently in the transmission channel.

### SUMMARY OF THE INVENTION

According to an aspect of the present disclosure, a controller for synchronizing image signals and sensor signals in a medical intervention includes a circuit that implements a process. The process implemented by the circuit includes receiving a signal stream between an ultrasound imaging probe that emits multiple beams during the medical intervention and a console that receives the image signals from the ultrasound imaging probe generated based on the multiple beams. The signal stream includes synchronization information indicating timing of emission of each beam of the multiple beams. The process implemented by the circuit also includes extracting, by the circuit from the signal stream, the synchronization information indicating the timing of emission of each beam of the multiple beams. The process implemented by the circuit further includes receiving, by the circuit from a first passive ultrasound sensor that receives energy from each beam emitted by the ultrasound imaging probe, a first signal that includes first sensor information indicative of a location of the first passive ultrasound sensor and generated based on receipt by the first passive ultrasound sensor of the energy received from each beam emitted by the ultrasound imaging probe. The process implemented by the circuit additionally includes adding to the first signal with the first sensor information and based on the synchronization information extracted from the signal stream, a second signal with a predefined signature characteristic indicating the timing of emission of each beam of the multiple beams, to produce a first combined signal. The process implemented by the circuit moreover includes sending, from the circuit to the console, the first combined signal produced by adding the first signal with the first sensor information and the second signal with the predefined signature characteristic indicating the timing of emission of each beam of the multiple beams.

According to another aspect of the present disclosure, a system for synchronizing image signals and sensor signals in a medical intervention includes an ultrasound imaging probe, a console, a first passive ultrasound sensor, and a controller. The ultrasound imaging probe emits multiple beams during the medical intervention. The console receives image signals from the ultrasound imaging probe generated based on the multiple beams. The first passive ultrasound sensor receives energy from each beam emitted by the ultrasound imaging probe. The controller includes a circuit that implements a process. The process implemented by the circuit includes receiving a signal stream between the ultrasound imaging probe and the console. The signal stream includes synchronization information indicating timing of emission of each beam of the multiple beams. The process implemented by the circuit also includes extracting, by the circuit from the signal stream, the synchronization information indicating the timing of emission of each beam of the multiple beams. The process implemented by the circuit further includes receiving, by the circuit from the first passive ultrasound sensor, a first signal that includes first sensor information indicative of a location of the first passive ultrasound sensor and generated based on receipt by the first passive ultrasound sensor of the energy received from each beam emitted by the ultrasound imaging probe. The process implemented by the circuit additionally includes adding to the first signal with the first sensor information and based on the synchronization information extracted from the signal stream, a second signal with a predefined signature characteristic indicating the timing of emission of each beam of the multiple beams, to produce a combined signal. The process implemented by the circuit moreover includes sending, from the circuit to the console, the combined signal produced by adding the first signal with the first sensor information and the second signal with the predefined signature characteristic indicating the timing of emission of each beam of the multiple beams.

According to another aspect of the present disclosure, a tangible non-transitory computer readable storage medium stores a computer program. When executed by a processor, the computer program causes a console to perform a process for synchronizing image signals and sensor signals in a medical intervention. The process performed when the processer executes the computer program includes receiving, from an ultrasound imaging probe, image signals generated based on multiple beams emitted by the ultrasound imaging probe. The process performed when the processor executes the computer program also includes receiving a first combined signal produced by adding a first signal with first sensor information and a second signal with a predefined signature characteristic indicating timing emission of each beam of the multiple beams. The first sensor information is indicative of a location of a first passive ultrasound sensor and is generated based on receipt of energy from each beam emitted by the ultrasound imaging probe by the first passive ultrasound sensor. The process performed when the processor executes the computer program further includes separating the first signal with the first sensor information from the second signal with the predefined signature characteristic. The process performed when the processor executes the computer program additionally includes obtaining, from the second signal, synchronization information indicating timing of emission of each beam of the multiple beams. The process performed when the processor executes the computer program moreover includes synchronizing, based on the synchronization information obtained from the second signal, images from the ultrasound imaging probe with sensor data of the first passive ultrasound sensor obtained from the first sensor information of the first signal.

According to another aspect not forming part of the current invention, a method for synchronizing images and sensor locations in a medical intervention includes receiving a signal stream between an ultrasound imaging probe that emits multiple beams during the medical intervention and a console that receives image signals from the ultrasound imaging probe generated based on the multiple beams. The signal stream includes synchronization information indicating timing of emission of each beam of the multiple beams. The method also includes extracting from the signal stream the synchronization information indicating the timing of emission of each beam of the multiple beams. The method further includes receiving from a first passive ultrasound sensor that receives energy from each beam emitted by the ultrasound imaging probe, a first signal that includes first sensor information indicative of a location of the first passive ultrasound sensor and generated based on receipt by the first passive ultrasound sensor of the energy received from each beam emitted by the ultrasound imaging probe. The method additionally includes adding to the first signal with the first sensor information and based on the synchronization information extracted from the signal stream, a second signal with a predefined signature characteristic indicating the timing of emission of each beam of the multiple beams, to produce a first combined signal. The method moreover includes sending to the console the first combined signal produced by adding the first signal with the first sensor information and the second signal with the predefined signature characteristic indicating the timing of emission of each beam of the multiple beams.

According to another aspect of the present disclosure, a computer program comprising computer readable code/instructions, which, when executed by a computer cause a console to perform the method as defined herein above which program may be stored on a non-transitory computer readable medium.

### BRIEF DESCRIPTION OF THE DRAWINGS

The example embodiments are best understood from the following detailed description when read with the accompanying drawing figures. It is emphasized that the various features are not necessarily drawn to scale. Thus, the dimensions may be arbitrarily increased or decreased for clarity of discussion. Wherever applicable and practical, like reference numerals refer to like elements.
FIG. 1 illustrates a system for encoded synchronized medical intervention image signals and sensor signals, in accordance with a representative embodiment.
FIG. 2 illustrates another system for encoded synchronized medical intervention image signals and sensor signals, in accordance with a representative embodiment.
FIG. 3 illustrates another system for encoded synchronized medical intervention image signals and sensor signals, in accordance with a representative embodiment.
FIG. 4 illustrates a method for another system for encoded synchronized medical intervention image signals and sensor signals.
FIG. 5 illustrates another method for another system for encoded synchronized medical intervention image signals and sensor signals.
FIG. 6 illustrates another method for another system for encoded synchronized medical intervention image signals and sensor signals.
FIG. 7 illustrates another method for another system for encoded synchronized medical intervention image signals and sensor signals.
FIG. 8 illustrates another method for another system for encoded synchronized medical intervention image signals and sensor signals.
FIG. 9 illustrates a general computer system, on which a method of encoded synchronized medical intervention image signals and sensor signals can be implemented, in accordance with another representative embodiment.

### DETAILED DESCRIPTION OF THE EMBODIMENTS

In the following detailed description, for purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In view of the foregoing, the present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

As described below, encoded synchronized medical intervention image signals and sensor signals can be used to encode the synchronization information from an ultrasound imaging system into a signal stream from a passive ultrasound sensor. This allows for variable downstream latency in the signal path without adversely affecting tracking accuracy and simplifies the simultaneous use of multiple passive ultrasound sensors with a single imaging probe. To be clear from the start, a passive ultrasound sensor may be considered a sensor that receives and detects energy from beams from an ultrasound imaging probe, and the receipt and detection may be considered passive in that the passive ultrasound sensor operates by not responding to the ultrasound imaging probe. Rather, the passive ultrasound sensor generates and sends a signal representative of the received energy and the underlying beams from the ultrasound imaging probe, and the signal sent by the passive ultrasound sensor can be processed by, for example, a console to determine the location of the passive ultrasound sensor.

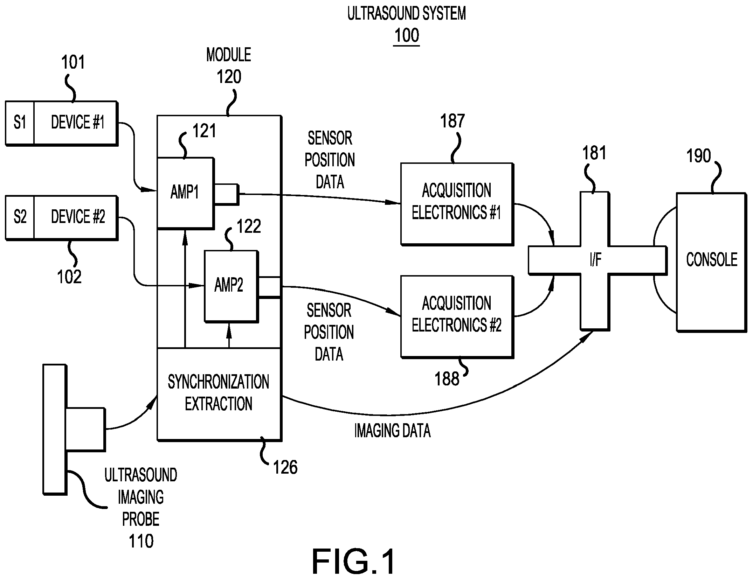

FIG. 1 illustrates a system for encoded synchronized medical intervention image signals and sensor signals, in accordance with a representative embodiment.

In FIG. 1, an ultrasound system 100 includes a first interventional medical device 101, a second interventional medical device 102, an ultrasound imaging probe 110, a module 120, a first acquisition electronics 187, a second acquisition electronics 188, an interface 181, and a console 190. A first passive ultrasound sensor S1 is placed on or in the first interventional medical device 101, and a second passive ultrasound sensor S2 is placed on or in the second interventional medical device 102.

Either or both of a first interventional medical device 101 and a second interventional medical device 102 may be a needle, a catheter, or another type of medical device inserted into a human subject during a medical procedure. The interventional aspect of the medical procedure may be considered the insertion of the interventional medical device into the human subject. The first passive ultrasound sensor S1 receives energy from each beam emitted by the ultrasound imaging probe 110. The second passive ultrasound sensor S2 also receives energy from each beam emitted by the ultrasound imaging probe 110. Of course, if the first passive ultrasound sensor S1 and/or the second passive ultrasound sensor S2 are not within the volume covered by a beam from the ultrasound imaging probe 110, then the first passive ultrasound sensor S1 and/or the second passive ultrasound sensor S2 will not receive the energy from the beam. However, when the first passive ultrasound sensor S1 is within the volume covered by a beam, the first passive ultrasound sensor S1 transmits a first signal that includes first sensor information indicative of a location of the first passive ultrasound sensor S 1. The first signal is generated based on receipt by the first passive ultrasound sensor S1 of the energy received from each beam emitted by the ultrasound imaging probe 110. Similarly, when the second passive ultrasound sensor S2 is within the volume covered by a beam, the second passive ultrasound sensor S2 transmits a third signal that includes second information indicative of a location of the second passive ultrasound sensor S2. The second signal is generated based on receipt by the second passive ultrasound sensor S2 of the energy received from each beam emitted by the ultrasound imaging probe 110.

The module 120 may be a controller for synchronizing image signals and sensor signals in a medical intervention. As a controller, the module 120 is or includes a circuit that implements or is used to implement some or all aspects of several processes described herein. The module 120 includes a first amplifier 121, a second amplifier 122, and a synchronization extraction sub-circuit 126. The first amplifier 121 amplifies a first analog signal received from the first interventional medical device 101. The second amplifier 122 amplifies a second analog signal received from the second interventional medical device 102. The first amplifier 121 and the second amplifier 122 are or may be located on a dongle between the first interventional medical device 101 (e.g., a needle) and the console 190. Alternatively, the first amplifier 121 and the second amplifier 122 may be in an enclosed module used for acquisition in the console 190.

In the module 120, the synchronization extraction sub-circuit 126 extracts timing information of each beam fired by the ultrasound imaging probe 110 from image signals received from the ultrasound imaging probe 110. To be clear, image signals received by the console 190 from the ultrasound imaging probe 110 may include images, timing information, element data, partially beamformed data, and any other image-related information sent from the ultrasound imaging probe 110 to the console 190. The images may be imagery from ultrasound imaging probes with beamformers integrated in the ultrasound imaging probes. The timing information may be information indicating the timing of emission of each beam emitted by the ultrasound imaging probe 110. The element data is the raw radio frequency (RF) data that is received by each individual transducer element, in the case for a simple ultrasound imaging probe 110 in which all beamforming takes place in the console 190. The partially beamformed data is initially beamformed (e.g., partially beamformed) in the ultrasound imaging probe 110 and further beamformed in the console 190 (such as for probes that have micro beamformers). The extracted timing information may therefore be only a subset of the image signals otherwise sent from the ultrasound imaging probe 110 to the console 190. Imaging data is passed from the synchronization extraction sub-circuit 126 to the interface 181. Additionally, the extracted timing information that is extracted by the synchronization extraction sub-circuit 126 is provided (e.g., sent, transmitted, passed) to the first amplifier 121 and the second amplifier 122 or separate elements connected in the path of the first analog signal from the first interventional medical device 101 and in the path of the second analog signal from the second interventional medical device 102.

The extracted timing information is synchronization information extracted from the signal stream from the ultrasound imaging probe 110 by the circuit in the module 120. As a controller, the module 120 implements the encoded synchronized medical intervention image signals and sensor signals described herein by adding to the first signal with the first sensor information and based on the synchronization information extracted from the signal stream from the ultrasound imaging probe 110, a second signal with a predefined signature characteristic indicating the timing of emission of each beam of the multiple beams emitted by the ultrasound imaging probe 110. The adding is performed to produce a first combined signal by the module 120, and particularly by a circuit of a controller in or as the module 120.

The adding performed by the circuit of the controller in the module 120 or as the module 120 may be performed in different ways depending on the nature of the predefined signature characteristic. For example, the adding by the circuit of the module 120 as a controller may include combining the first signal from the first passive ultrasound sensor S1 with a predefined waveform as the predefined signature characteristic. As a result, the console 190 can detect the predefined waveform in the first combined signal. In another example, the adding by the circuit of the module 120 as a controller may include combining the first signal from the first passive ultrasound sensor S1 with a first predefined waveform and a second predefined waveform as the predefined signature characteristic. As a result, the console 190 can detect the first predefined waveform and the second predefined waveform in the first combined signal. The first predefined waveform corresponds to or may correspond to a frame trigger. The second predefined waveform corresponds to or may correspond to a line trigger. In another example, the adding may include combining the first signal from the first passive ultrasound sensor S1 with a positive voltage pulse as the predefined signature characteristics. As a result, the console 190 can detect the positive voltage pulse in the first combined signal.

While the adding described above is limited to the first signal from the first passive ultrasound sensor S 1, the same adding may be performed for a third signal from the second passive ultrasound sensor S2. The module 120 (e.g., the second amplifier 122) may perform adding by adding a predefined waveform, a first predefined waveform, a second predefined waveform and/or a positive voltage pulse to the third signal from the second passive ultrasound sensor S2. As a result, the console 190 can detect the predefined waveform, the first predefined waveform, the second predefined waveform, and/or the positive voltage pulse in a second combined signal.

In the embodiment of FIG. 1, the signal stream received by the module 120 in order to extract the synchronization information indicating the timing of emission of each beam by the ultrasound imaging probe 110 is received from the ultrasound imaging probe 110. That is, the ultrasound imaging probe 110 sends information indicating the timing of emitting each beam along with the imagery captured as a result of the beams by the ultrasound imaging probe 110. The ultrasound imaging probe 110 may also send other information in the image signals of the signal stream to the console 190. For example, the signal stream may include identification of beam number of each beam, and even characteristics of each beam, along with the timing of emission, and imagery captured by the ultrasound imaging probe 110. Additionally, the various kinds of information present in the image stream from the ultrasound imaging probe 110 may not all correspond to the same beam. Rather, at one time or in one packet or in one burst the ultrasound imaging probe 110 may include the most recently captured images along with identification of a beam most recently fired for which images have not yet been captured. Thus, the timing of emission of each beam indicated by the synchronization information in the signal stream may be extracted from images or other information that are not directly correlated with the beam indicated by the synchronization information. Thus, offsets may be present in the signal stream such as between groupings of image(s) sent at one time and other information such as synchronization information sent with the image(s) at the same time.

The first acquisition electronics 187 receives the first combined signal from the module 120, such as from the first amplifier 121. The first combined signal includes the sensor position data of the first passive ultrasound sensor S 1 on, in or with the first interventional medical device 101. The first acquisition electronics 187 passes the first combined signal to the interface 181, and the interface 181 passes the first combined signal to the console 190. The first acquisition electronics 187, the interface 181 or the console 190 may detect the predefined signature characteristic indicating the timing of emission of each beam from the first combined signal and may extract the timing of emission of each beam. As a result, the location of the first passive ultrasound sensor S1 may be displayed on a display tied to the console 190 at a timing correlated properly with the images received via the imaging data from the ultrasound imaging probe 110, even if the timing of emission of each beam and the images received via the imaging data are offset from another in the signal stream.

Additionally, the second acquisition electronics 188 receives the second combined signal from the module 120, such as from the second amplifier 122. The second combined signal includes the sensor position data of the second passive ultrasound sensor S2 on, in or with the second interventional medical device 102. The second acquisition electronics 188 passes the second combined signal to the interface 181, and the interface 181 passes the second combined signal to the console 190. The second acquisition electronics 188, the interface 181 or the console 190 may detect the predefined signature characteristic indicating the timing of emission of each beam from the second combined signal and may extract the timing of emission of each beam. As a result, the location of the second passive ultrasound sensor S2 may be displayed on a display tied to the console 190 at a timing correlated properly with the images received via the imaging data from the ultrasound imaging probe 110, even if the timing of emission of each beam and the receipt of images via the imaging data are offset from one another in the signal stream. Therefore, the console 190 or a device or system tied to the console 190 generates or may generate a display of images from the image signals from the ultrasound imaging probe 110 and the location of the first passive ultrasound sensor S1 synchronized based on the predefined signature characteristic.

The interface 181 may include software that implements interface protocols, hardware such as ports, and any other components that enable the first acquisition electronics 187 and the second acquisition electronics 188 to interface with the console 190. The console 190 may include a memory that stores instructions, a processor that executes the instructions, and other circuit elements appropriate to implement some or all aspects of processes attributed herein to a console 190. For example, the console 190 may include input mechanisms such as a keyboard, mouse and/or touchscreen, and display mechanisms such as a video screen to display ultrasound images, sensor locations, and other information received by the console 190.

To ensure consistency with the flows shown in FIG. 1, the ultrasound imaging probe 110 sends a signal stream that is received by the circuit of the module 120 acting as a controller. The circuit of the module 120 extracts the synchronization information from the signal stream. The first passive ultrasound sensor S1 and the second passive ultrasound sensor S2 also send the first signal and the third signal that are received by the circuit of the module 120 acting as a controller. The second signal with the predefined signature characteristic is added to the first signal and the third signal by the circuit of the module 120 acting as a controller, based on the synchronization information extracted by the circuit of the module 120 from the signal stream. The first combined signal and the second combined signal each include sensor position data of the corresponding passive ultrasound sensor and are each sent by the circuit of the module 120 acting as a controller to the console 190 via the first acquisition electronics 187 and the second acquisition electronics 188 and the interface 181. The console 190 or the interface 181 can detect any offset between the underlying synchronization information and any corresponding ultrasound imagery received with the synchronization information, so that ultrasound imagery displayed by a display is aligned based on the offset (if any) to properly show the location of the first passive ultrasound sensor S1 and/or the location of the second passive ultrasound sensor S2.

In the embodiment of FIG. 1 described above, the ultrasound imaging probe 110 and the first passive ultrasound sensor S1 and the second passive ultrasound sensor S1 are connected to the module 120 placed near the patient. The module 120 is also connected to the console 190, such that the ultrasound imaging data stream from the ultrasound imaging probe 110 is passed through the module 120 and the amplified sensor signals are transmitted to the console 190. The module 120 contains a circuit that extracts the synchronization signals from the signal stream from the ultrasound imaging probe 110. The extracted synchronization signals are or may be used to initiate the injection of predefined signal wave forms into the sensor amplifier path. As a result, the same communication line that carries a sensor signal also has a synchronization signal on it. At the console 190, this analog signal is or may be digitized and the location of the predefined signal waveform is or may be detected, so the console 190 can determine the timing between the sensor signal and the acoustic transmit events. The embodiment of FIG. 1 is readily applicable to both high end platforms that have beamforming in the console 190, and value segment platforms that beamform in the ultrasound imaging probe 110 and send a digital signal to the console 190.

The predefined signal waveforms in the embodiment of FIG. 1 and other embodiments herein may take many shapes. One important criteria for such predefined signal waveforms is that the waveform shape be sufficiently different from any possible sensor signal to avoid ambiguity. Two or more distinct waveform shapes are needed to separate a frame trigger and a line trigger. An example of the waveform shapes described herein includes a positive voltage injected into the amplifier chain to cause the amplifier to saturate at its positive supply voltage. The width of such a square pulse may be made wider than the period of the lowest frequency components expected from the sensor signal, and pulse width modulation may be used to distinguish frame trigger and line trigger. The position of the rising edge may be used to indicate the time the transmit event took place. Another example of the waveform shapes described herein is a sequence of positive and negative pulses forming, for example, a 16 bit code. The code may be used to indicate the beam number. The synchronization extraction circuit may also extract additional information such as imaging mode, and this information could be included in a 16 bit code used as the waveform shapes. To be clear, the waveform shapes described herein are the predefined signature characteristic explained elsewhere in this disclosure.

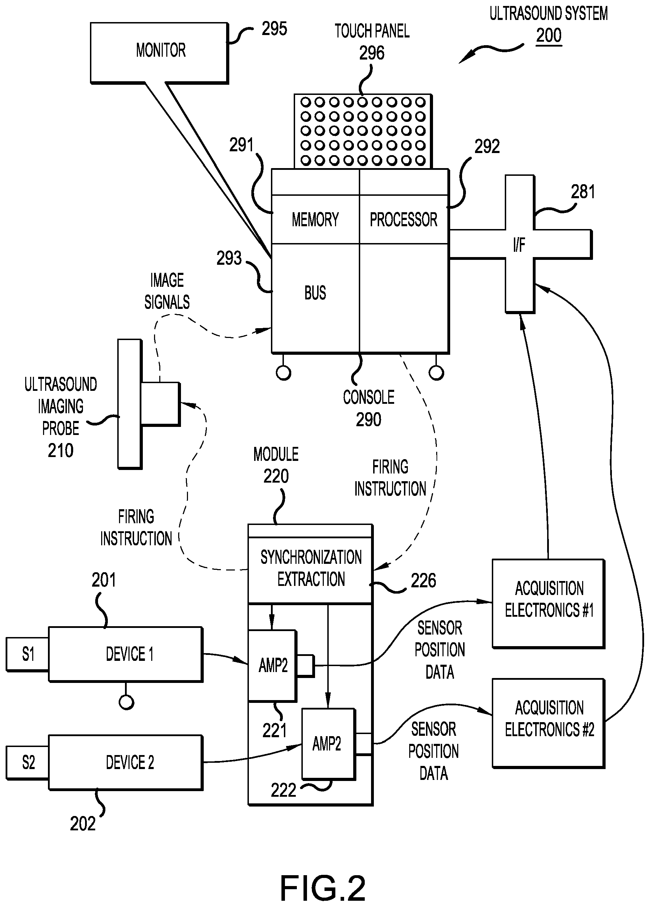

FIG. 2 illustrates another system for encoded synchronized medical intervention image signals and sensor signals, in accordance with a representative embodiment.

In FIG. 2, an ultrasound system 200 includes a first interventional medical device 201, a second interventional medical device 202, an ultrasound imaging probe 210, a module 220, a first acquisition electronics 287, a second acquisition electronics 288, an interface 281, and a console 290. A first passive ultrasound sensor S1 is placed on or in the first interventional medical device 201, and a second passive ultrasound sensor S2 is placed on or in the second interventional medical device 202. In the embodiment of FIG. 2, elements with names and numbers corresponding to elements in the embodiment of FIG. 1 may be the same or similar, with differences as described herein. One notable difference between the features in Fig. 1 and the features in FIG. 2 is that the console 290 in FIG. 2 is broken out to include a memory 291, a processor 292, and a bus 293. Additionally, in the embodiment of FIG. 2 a monitor 295 and a touch panel 296 are connected to the console 290, such as in a dedicated relationship. To be clear, however, the console 190 in the embodiment of FIG. 1 may also have a monitor and/or a touch panel connected thereto, such as in a dedicated relationship. Thus, the components of the ultrasound system 200 which are not shown in the ultrasound system 100 may still be present in the ultrasound system 100 unless otherwise indicated herein.

The module 220 may be a controller for synchronizing image signals and sensor signals in a medical intervention. As a controller, the module 220 is or includes a circuit that implements or is used to implement some or all aspects of several processes described herein. The module 220 includes a first amplifier 221, a second amplifier 222, and a synchronization extraction sub-circuit 226.

The expanded console 290 in the embodiment of FIG. 2 is provided to explain that the signal stream in the embodiment is from the console 290 to the ultrasound imaging probe 210 via the circuit of the module 220 acting as a controller. As a reminder, in the embodiment of FIG. 1 above, the signal stream was from the ultrasound imaging probe 110 to the console 190 via the circuit of the module 120 acting as a controller. In FIG. 2, the console 290 may implement the firing instructions to instruct the ultrasound imaging probe 210 when to fire a beam and what to fire as a beam. For example, the console 290 may execute instructions from the memory 291 by the processor 292 in order to identify and implement a pattern of imaging beams via the ultrasound imaging probe 210. For example, a user may input a pattern of imaging beams to fire via the console 290, so that the console 290 provides firing instructions for each beam, either individually, in groups, or in a complete set in advance. No matter how the console 290 sends the firing instructions to the ultrasound imaging probe 210 in FIG. 2, the module 220 can extract the synchronization information from the signal stream to the ultrasound imaging probe 210 and add the second signal with the predefined signature characteristic to the first signal with the first sensor information. As a result, a first combined signal is produced for the sensor position of the first passive ultrasound sensor S1 and a second combined signal is produced for the sensor position of the second passive ultrasound sensor S2. The first combined signal includes sensor position data of the first passive ultrasound sensor S1 with the predefined signature characteristic, and the second combined signal includes sensor position data of the second passive ultrasound sensor S2 along with the added second signal with the predefined signature characteristic. As a result, the console 290 can detect and extract the predefined signature characteristic, apply any appropriate offset, and align the locations of the first passive ultrasound sensor S1 and the second passive ultrasound sensor S2 with the ultrasound images corresponding to the time at which the locations are captured.

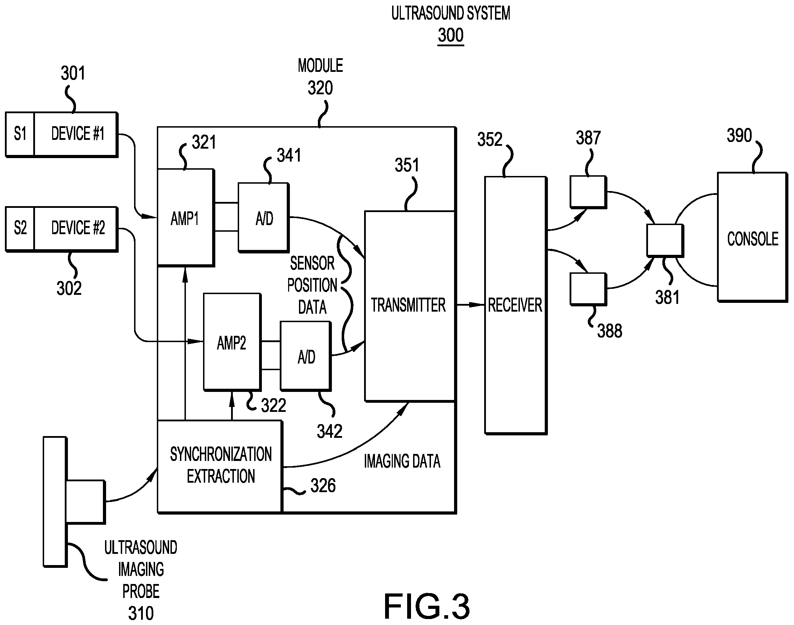

FIG. 3 illustrates another system for encoded synchronized medical intervention image signals and sensor signals, in accordance with a representative embodiment.

In FIG. 3, an ultrasound system 300 includes a first interventional medical device 301, a second interventional medical device 302, an ultrasound imaging probe 310, a module 320, a first acquisition electronics 387, a second acquisition electronics 388, an interface 381, and a console 390. A first passive ultrasound sensor S1 is placed on or in the first interventional medical device 301, and a second passive ultrasound sensor S2 is placed on or in the second interventional medical device 302. In the embodiment of FIG. 3, elements with names and numbers corresponding to elements in the embodiment of FIG. 1 and/or the embodiment of FIG. 2 may be the same or similar, with differences as described herein. One notable difference between the features in Figs. 1 and FIG. 2 and the features in FIG. 3 is that the module 320 is broken out to include a first amplifier 321, a second amplifier 322, a first analog-to-digital converter 341, a second analog-to-digital converter 34, a transmitter 351, and a synchronization extraction sub-circuit 326. To be clear, however, the module 120 in the embodiment of FIG. 1 and the module 220 in the embodiment of FIG. 2 may also have analog-to-digital converters and transmitters. Thus, the components of the ultrasound system 300 which are not shown in the ultrasound system 100 or the ultrasound system 200 may still be present in the ultrasound system 100 and/or the ultrasound system 200 unless otherwise indicated herein.

In the embodiment of FIG. 3, the circuit of the module 320 acting as a controller digitizes the first signal from the first passive ultrasound sensor S1 by the first analog-to-digital converter 341. The circuit of the module 320 acting as a controller also digitizes the third signal from the second passive ultrasound sensor S2 by the second analog-to-digital converter 342. Accordingly, in the embodiment of FIG. 3 a process implemented by the circuit of the module 320 acting as a controller includes digitizing, by the circuit, the amplified first signal from the first amplifier 321 to produce the first signal. The adding then includes combining the first signal with the predefined signature characteristic. For example, the adding may include combining the digitized and amplified first signal with at least one pulse representing at least one digital bit as the predefined signature characteristic.

The digitizing by the circuit of the module 320 in the embodiment of FIG. 3 may be used for numerous different ends. For example, a process implemented by the circuit of the module 320 as a controller may include receiving the output from the first passive ultrasound sensor S 1 as a first sensor output, digitizing, by the circuit, the first sensor output to produce a digitized sensor output, and digitizing, by the circuit, the signal stream (from the ultrasound imaging probe 310) to produce a digitized signal stream. The process may also include combining the digitized signal stream and the digitized sensor output to produce a digitized first combined signal as the first combined signal provided to the console 390. Incidentally, an analog-to-digital converter is not shown for the signal stream from the ultrasound imaging probe 310, but one can be provided in the module 320 between the synchronization extraction sub-circuit 326 and the transmitter 351 to digitize the imaging data before transmission by the transmitter 351.

One aspect of the digitization in the embodiment of FIG. 3 is that the module 320 also includes a transmitter 351 that can be used to transmit the digitized first combined signal to a receiver 352. Of course, the transmitter 351 can also be used to transmit a second combined signal to the receiver 352 based on the third signal from the second passive ultrasound sensor S2 as modified by the adding of the predefined signature characteristic before the conversion by the second analog-to-digital converter 342. A process implemented by the circuit of the module 320 acting as a controller may therefore include transmitting, by the circuit, a digitized first combined signal from the transmitter 351 for receipt by a receiver 352 that interfaces with the console 390.

The embodiment of FIG. 3 is applicable, for example, to imaging platforms that have beamforming in the ultrasound imaging probe 310 and are sending a digital signal to the console 390. The ultrasound imaging probe 310 and the first passive ultrasound sensor S1 and the second passive ultrasound sensor S2 are connected to the module 320 placed near the patient. The probe signal is routed through a synchronization extraction sub-circuit 326 to extract the timing of the acoustic transmit events. The sensor signals are amplified and digitized, and the timing information is added to the digital data stream of each sensor signal. The resultant data streams are digital, one for the ultrasound image and one for each sensor. These digital data streams can be merged into one big digital data stream that is transmitted by the transmitter 351.

As an example, one digital USB-2 stream may contain the ultrasound imaging data. This USB-2 stream may be combined with the digital data streams from the sensors and converted into a USB-3 protocol in the transmitter. A USB-3 cable may be used to connect the transmitter 351 to the receiver 352 in or at the console 390. Alternatively, the transmission may be a wireless transmission. As another alternative, the data streams may be converted into a Gigabit Ethernet data stream.

The data transmission protocols between transmitter 351 in or at the module 320 and the receiver 352 in or at the console 390 may divide the data stream into packets, and the different packets experience a varying degree of latency. Encoding the synchronization signal in the sensor digital data stream creates an advantage, as even if packets arrive out of order the timing of the transmit event relative to the sensor signal can still be fully reconstructed.

As described herein, there are many ways to inject the synchronization signal into the sensor digital data stream. For example, with a 14 bit A/D converter and a 16 bit data stream, the least significant bits may be used for the digitized sensor signal and the upper two bits for synchronization data. As a specific example, digits 00 may indicate no synchronization, 01 may indicate a frame trigger, and 10 may indicate a line trigger. In another embodiment, an analog waveform may be injected into the amplifier, as in the embodiment of FIG. 1, or a digital waveform may be injected directly after the A/D conversion.

The sensor signal digitization may be started at the moment a synchronization signal is extracted and may have a duration set by the imaging depth. A header with synchronization information may be pre-appended to the data stream. In this situation it may also be possible to reduce the amount of data that needs to be transferred, passive ultrasound sensors use only one way sound travel and the sensor signal needs to only be sampled 50% of the time. A device ID may also be included in the header, so that the console 390 is able to differentiate multiple simultaneously tracked devices.

In FIG. 3 the only connection between the transmitter 351 in or at the module 320 and the receiver 352 in the console 390 may be a digital communication channel. As a result, the console 390 may be implemented in numerous different ways. For example, the console 390 may be embedded into a visualization system, or an augmented reality system based on for example a holographic system.

In embodiments, the communication channel in FIG. 3 may be the internet and multiple consoles such as the console 390 may be involved. For example, an interventionalist at one location with the console 390 may be coordinated with a wireless console that only visualizes the ultrasound and device tracking data. At a completely different location an expert/assistant with another console similar or identical to the console 390 may be used for visualization and manipulating imaging settings. When the communication channel can produce latency variations in excess of an imaging frame, a frame number may be added in the headers of both the sensor data stream and the imaging data stream.

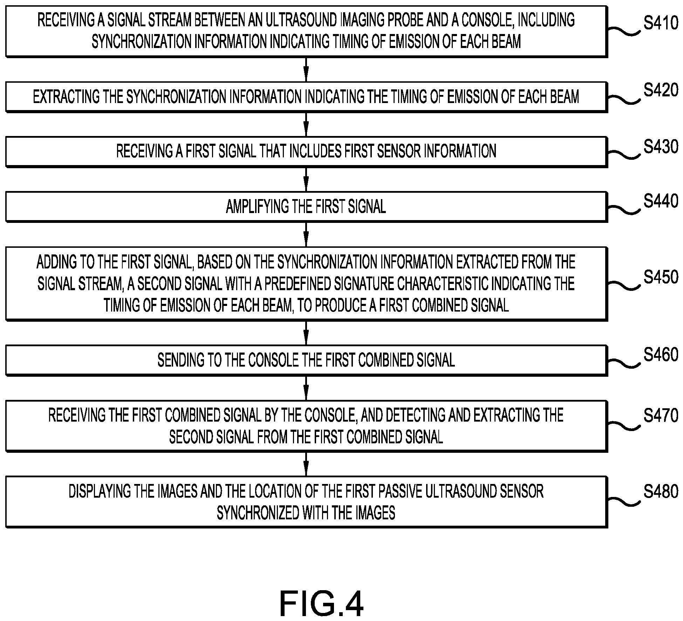

FIG. 4 illustrates a method for another system for encoded synchronized medical intervention image signals and sensor signals.

The method in FIG. 4 is a method for synchronizing image signals and sensor signals in a medical intervention. The method in FIG. 4 starts at S410 with receiving a signal stream between an ultrasound imaging probe and a console, including synchronization information indicating timing of emission of each beam. The signal stream can be received by the module 120, the module 220, or by the module 320. The signal stream can be received from the ultrasound imaging probe 110 or the ultrasound imaging probe 310 as in the embodiments of FIGs. 1 and 3, or from the console 290 as in the embodiment of FIG. 2. The signal stream may therefore include images, beam sequence and timing of firing of each beam as in the embodiments of FIGs. 1 and 3, or simply firing instructions (which may reflect a beam sequence too) as in the embodiment of FIG. 2.

The method in FIG. 4 continues at S420 with extracting the synchronization information indicating the timing of emission of each beam. The extracting at S420 is performed by the synchronization extraction sub-circuit 126, the synchronization extraction sub-circuit 226, or the synchronization extraction sub-circuit 326. The extracting may be based on recognizing characteristics of the synchronization information, such as at periodic timings in the signal stream, based a pattern used to mark the synchronization information, a bandwidth used to carry the synchronization information, and/or other characteristics.

The method in FIG. 4 proceeds again at S430 with receiving a first signal that includes first sensor information. The first signal is received at S430 by the module 120, the module 220 or the module 320, and specifically by the first amplifier 121, the first amplifier 221 or the first amplifier 321. Alternatively, the receiving at S430 may be performed by another element (not shown) that performs the receiving before providing the first combined signal to the first amplifier 121, the first amplifier 221 or the first amplifier 321.

The method in FIG. 4 next proceeds at S440 with amplifying the first signal. The first signal is amplified at S440 by the module 120, the module 220 or the module 320, and again specifically by the first amplifier 121, the first amplifier 221 or the first amplifier 321.

Next, the method in FIG. 4 continues at S450 with adding to the first signal, based on the synchronization information extracted from the signal stream, a second signal with a predefined signature characteristic indicating the timing of emission of each beam, to produce a first combined signal. The adding may be performed at S450 by simply combining the two signals so that the resultant first combined signal has the sum of the amplitudes of the two signals at each component of the frequency spectrum. The adding is performed by the module 120, the module 220, or the module 320. The adding may be specifically performed by the first amplifier 121, the first amplifier 221 or the first amplifier 321. Alternatively, the adding at S450 may be performed by another element (not shown) that performs the adding before providing the first combined signal to the first amplifier 121, the first amplifier 221 or the first amplifier 321.

Although the adding at S450 is shown after the amplifying at S440, the adding may be performed before the amplifying at S440, such as by another element that performs the receiving at S430 in the module S120, the module S220 or the module S320. Additionally, the receiving at S430, the amplifying at S440 and the adding at S450 are explained in the context of a first signal that includes first sensor information for the first passive ultrasound sensor S1. However, the receiving at S430, the amplifying at S440 and the adding at S450 are also performed or may also be performed in parallel for the third signal that includes second sensor information for the second passive ultrasound sensor S2.

At S460, the method in FIG. 4 next includes sending to the console the first combined signal. The sending at S460 may be performed by the module 120, the module 220 or the module 320, and specifically by the first amplifier 121, the first amplifier 221 or the transmitter 351. Additionally, the sending at S460 is via intermediate elements such as the first acquisition electronics 187 and the interface 181, via the first acquisition electronics 287 and the interface 281, or via the receiver 352, the first acquisition electronics 387 and the interface 381. Moreover, the sending at S460 is performed in parallel for the second combined signal based on the third signal that includes second sensor information for the second passive ultrasound sensor S2.

The method in FIG. 4 again proceeds at S470 with receiving the first combined signal by the console and detecting and extracting the second signal from the first combined signal. The receiving at S470 is performed by the console 190, the console 290 or the console 390, and is via the interface 181, the interface 281 or the interface 381. The receiving at S470 is explained with respect to the first combined signal but is or may also be performed in parallel with respect to the second combined signal.

At S480 the method shown in FIG. 4 concludes with displaying the images and the location of the first passive ultrasound sensor synchronized with the images. The displaying at S480 is performed by or using the console 190, the console 290 or the console 390. For example, any of these consoles may be connected to a display such as a monitor and may control such a monitor to display the images and the location of the first passive ultrasound sensor synchronized with the images. For example, the location of the first passive ultrasound sensor may be superimposed on images in synchronization that is based on the synchronization information extracted at S420. Additionally, the displaying at S480 is explained with respect to the first passive ultrasound sensor S1 but is or may also be performed in parallel with respect to the second passive ultrasound sensor S2.

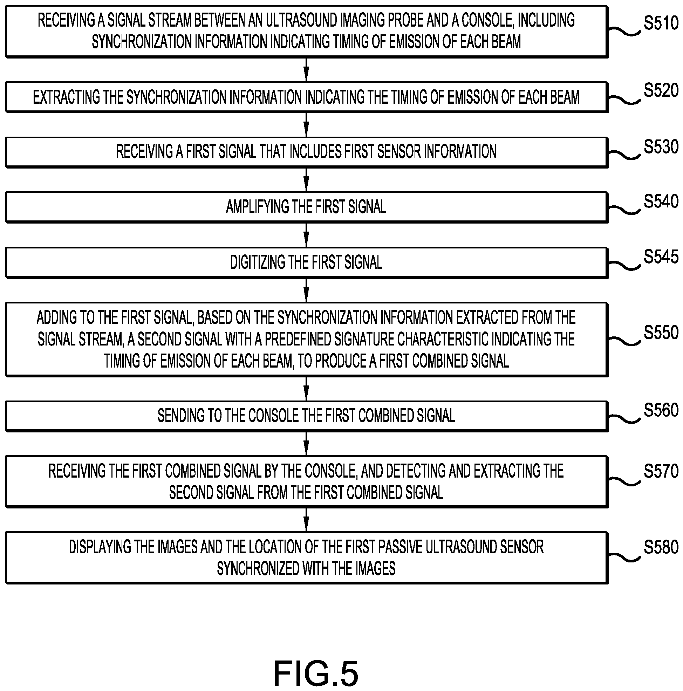

FIG. 5 illustrates another method for another system for encoded synchronized medical intervention image signals and sensor signals.

The method in FIG. 5 is another method for synchronizing image signals and sensor signals in a medical intervention, and largely overlaps the method in FIG. 4 with variations explained below. That is, the method in FIG. 5 starts at S5 10 with receiving a signal stream between an ultrasound imaging probe and a console, including synchronization information indicating timing of emission of each beam. The method in FIG. 5 continues at S520 with extracting the synchronization information indicating the timing of emission of each beam. The method in FIG. 5 proceeds again at S530 with receiving a first signal that includes first sensor information. The method in FIG. 5 next proceeds at S540 with amplifying the first signal.

In a variation from the method in FIG. 4, the method in FIG. 5 includes digitizing the first signal at S545. The digitizing at S545 may be also performed in parallel for the third signal and can be used for a variety of purposes such as to add synchronization information as digitized input to the digitized first signal.

Next, the method in FIG. 5 continues at S550 with adding to the first signal, based on the synchronization information extracted from the signal stream, a second signal with a predefined signature characteristic indicating the timing of emission of each beam, to produce a first combined signal. Given the digitization at S545, the adding at S550 may be digital adding of logical inputs rather than combining two signals so that the resultant first combined signal has the sum of the amplitudes of the two signals at each component of the frequency spectrum as explained previously for the embodiment of FIG. 4. The adding at S450 or at S550 may also include other forms of adding as explained herein.

At S560, the method in FIG. 5 next includes sending to the console the first combined signal. The method in FIG. 5 again proceeds at S570 with receiving the first combined signal by the console and detecting and extracting the second signal from the first combined signal. At S580 the method shown in FIG. 5 concludes with displaying the images and the location of the first passive ultrasound sensor synchronized with the images.

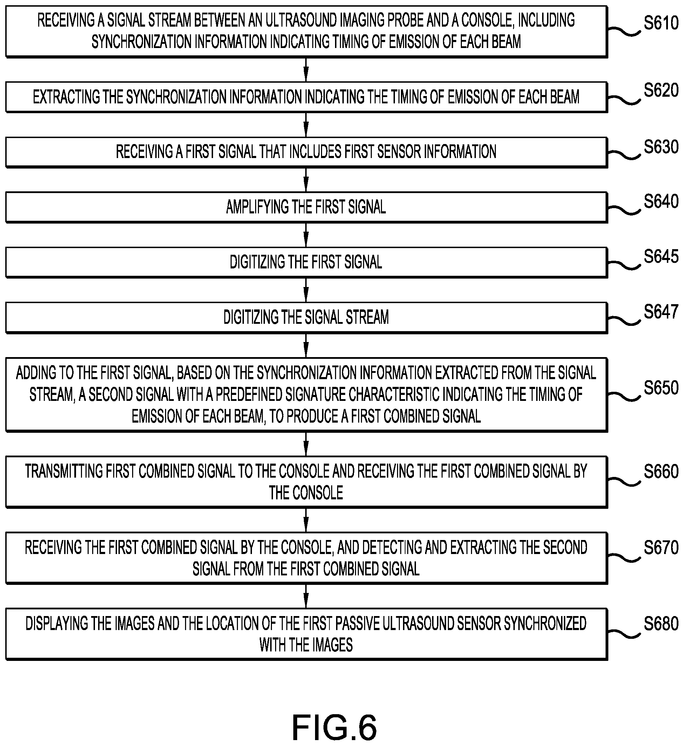

FIG. 6 illustrates another method for illustrates another system for encoded synchronized medical intervention image signals and sensor signals.

The method in FIG. 6 is another method for synchronizing image signals and sensor signals in a medical intervention, and largely overlaps the methods in FIG. 4 and in FIG. 5 with variations explained below. That is, the method in FIG. 6 starts at S610 with receiving a signal stream between an ultrasound imaging probe and a console, including synchronization information indicating timing of emission of each beam. The method in FIG. 6 continues at S620 with extracting the synchronization information indicating the timing of emission of each beam. The method in FIG. 6 proceeds again at S630 with receiving a first signal that includes first sensor information. The method in FIG. 6 next proceeds at S640 with amplifying the first signal.

In a variation from the method in FIG. 4, the method in FIG. 6 also includes digitizing the first signal at S645. The digitizing at S645 may be similar or the same to the digitizing explained above for S545 in the embodiment of FIG. 5.

In a variation from the method in FIG. 4 and the method in FIG. 5, the method in FIG. 6 also includes digitizing the signal stream at S647. That is, the signal stream from the ultrasound imaging probe 110, the ultrasound imaging probe 210 or the ultrasound imaging probe 310 may be digitized by an analog-to-digital converter not shown in the embodiments of FIGs. 1-3. The digitization at S647 may be performed for any of multiple different reasons, such as to make the signal stream compatible with the digitized first signal and/or to prepare the signal stream for transmission by the transmitter 351 in the embodiment of FIG. 3.

Next, the method in FIG. 6 continues at S650 with adding to the first signal, based on the synchronization information extracted from the signal stream, a second signal with a predefined signature characteristic indicating the timing of emission of each beam, to produce a first combined signal.

In another variation from the method in FIG. 4 and the method in FIG. 5, the method in FIG. 6 next proceeds at S660 with transmitting the first combined signal to the console and receiving the first combined signal by the console. The transmission at S660 may be performed by the transmitter 351 in the embodiment of FIG. 3, and the receiving may be performed via an intermediary for the console such as by the receiver 352 in the embodiment of FIG. 3. To be clear, the transmission at S660 may be over a long distance, and this is an example of how an unknown latency could be introduced but for the extraction of the synchronization information and related processes and features described herein.

The method in FIG. 6 again proceeds at S670 with receiving the first combined signal by the console and detecting and extracting the second signal from the first combined signal. At S680 the method shown in FIG. 6 concludes with displaying the images and the location of the first passive ultrasound sensor synchronized with the images.

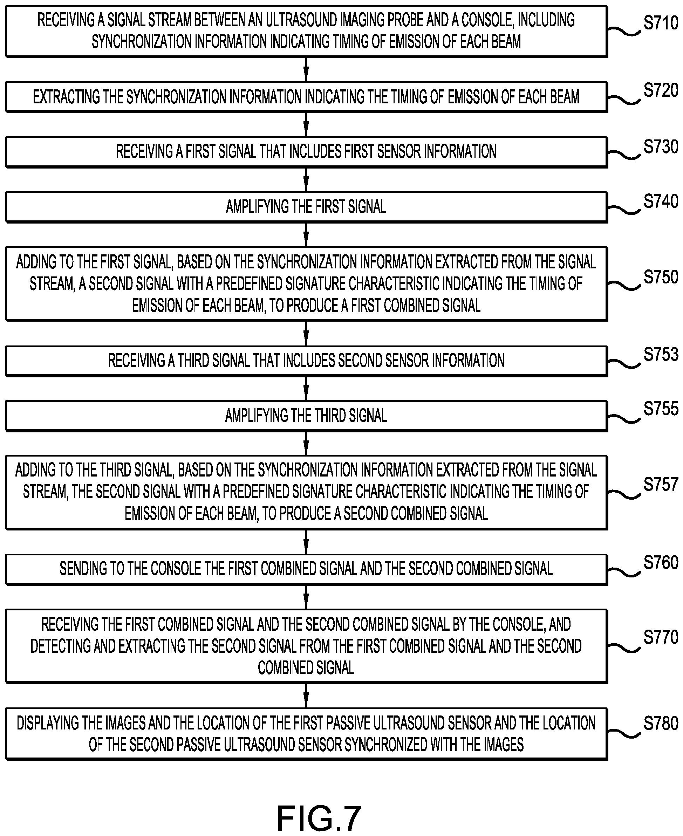

FIG. 7 illustrates another method for another system for encoded synchronized medical intervention image signals and sensor signals.

The method in FIG. 7 is another method for synchronizing image signals and sensor signals in a medical intervention, and largely overlaps the methods in FIG. 4, in FIG. 5 and in FIG. 6 with variations explained below. That is, the method in FIG. 7 starts at S710 with receiving a signal stream between an ultrasound imaging probe and a console, including synchronization information indicating timing of emission of each beam. The method in FIG. 7 continues at S720 with extracting the synchronization information indicating the timing of emission of each beam. The method in FIG. 7 proceeds again at S730 with receiving a first signal that includes first sensor information. The method in FIG. 7 next proceeds at S740 with amplifying the first signal. Next, the method in FIG. 7 continues at S750 with adding to the first signal, based on the synchronization information extracted from the signal stream, a second signal with a predefined signature characteristic indicating the timing of emission of each beam, to produce a first combined signal.

In a variation from the method in FIG. 4, the method in FIG. 5 and the method in FIG. 6, the method in FIG. 7 next includes receiving a third signal that includes second sensor information. That is, the embodiment of FIG. 7 formally shows the processes for the third signal that includes second sensor information for the second passive ultrasound sensor S2. The embodiments of FIGs. 4-6 may also include some or all of the features for the second sensor information unless otherwise specified.

In another variation from the method in FIG. 4, the method in FIG. 5 and the method in FIG. 6, the method in FIG. 7 also includes amplifying the third signal at S755. The amplifying of the third signal at S755 may be performed by the second amplifier 122, the second amplifier 222 or the second amplifier 322.

In yet another variation from the method in FIG. 4, the method in FIG. 5 and the method in FIG. 6, the method in FIG. 7 at S757 includes adding to the third signal, based on the synchronization information extracted from the signal stream, the second signal with a predefined signature characteristic indicating the timing of emission of each beam, to produce a second combined signal. To be clear, the predefined signature characteristic added to the third signal at S757 may be the same predefined signature characteristic added to the first signal at S750. Thus, the synchronization is performed in the same way for different signals from different passive ultrasound sensors.

In still another variation from the method in FIG. 4, the method in FIG. 5 and the method in FIG. 6, the method in FIG. 7 next proceeds at S760 with sending to the console the first combined signal and the second combined signal.

At S770, the method in FIG. 7 again diverges from earlier-described methods by receiving the first combined signal and the second combined signal by the console and detecting and extracting the second signal from both of the first combined signal and the second combined signal.

At S780, the method in FIG. 7 includes displaying the images and the location of the first passive ultrasound sensor and the location of the second passive ultrasound sensor synchronized with the images. As a result, a display tied to the console 190, the console 290 or the console 390 may display locations of both the first passive ultrasound sensor S 1 and the second passive ultrasound sensor S2 synchronized with the proper ultrasound images and in the accurate locations relative to the ultrasound images.

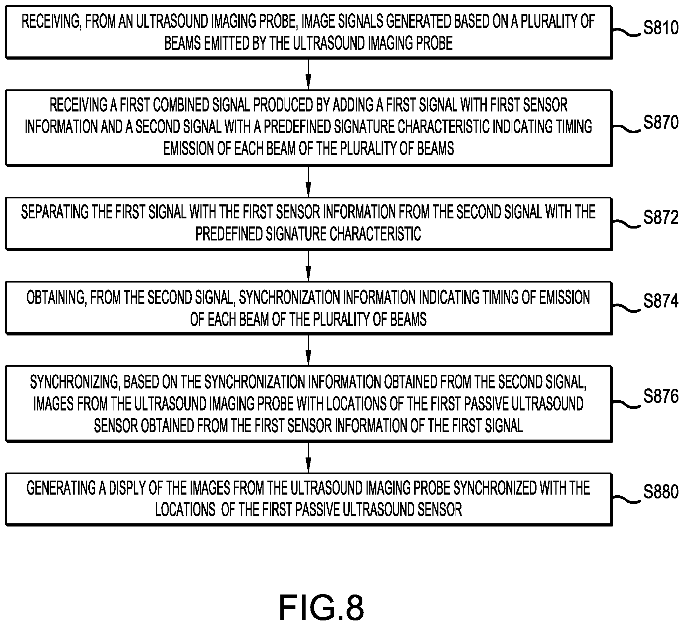

FIG. 8 illustrates another method for another system for encoded synchronized medical intervention image signals and sensor signals.

The method in FIG. 8 may be a method performed by a console as described herein, for example. The method in FIG. 8 starts at S810 by receiving, from an ultrasound imaging probe, image signals generated based on multiple beams emitted by the ultrasound imaging probe.

Next, the method in FIG. 8 includes receiving at S870 a first combined signal produced by adding a first signal with a first sensor information and a second signal with a predefined signature characteristic indicating timing emission of each beam of the multiple beams. That is, the console 190, the console 290 or the console 390 receives the first combined signal at S870. Of course, the second combined signal may also be received in parallel at S870.

At S872, the method in FIG. 8 includes separating the first signal with the first sensor information from the second signal with the predefined signature characteristic. The separating at S872 may also include separating the third signal with the second sensor information from the second signal with predefined signature characteristic in parallel. The separating may be performed for an analog signal as in the embodiments of FIGs. 1-2, or for a digitized signal as in the embodiment of FIG. 3.

At S874, the method in FIG. 8 next includes obtaining, from the second signal, synchronization information indicating timing of emission of each beam of the multiple beams.

At S876, the method in FIG. 8 proceeds to synchronizing, based on the synchronization information obtained from the second signal, images from the ultrasound imaging probe with locations of the first passive ultrasound sensor obtained from the first sensor information of the first signal. The synchronizing at S876 may include applying an offset in order to match the locations of the passive ultrasound sensors with the proper images in the event that the images are not received at the same time as the information of the locations of the passive ultrasound sensors, and this may be performed using the synchronization information described herein.

At S880, the method in FIG. 8 concludes with generating a display of the images from the ultrasound imaging probe synchronized with the locations of the first passive ultrasound sensor S1. The display generated at S880 may include generating a display of the locations of the second passive ultrasound sensor S2 in parallel with the display of the locations of the first passive ultrasound sensor S 1. Therefore, when executed by a processor, a computer program causes or may cause a console (e.g., the console 390) to generate a display of the images from the ultrasound imaging probe (e.g., the ultrasound imaging probe 310) synchronized with locations of the first passive ultrasound sensor S1 and/or synchronized with locations of the second passive ultrasound sensor S2.

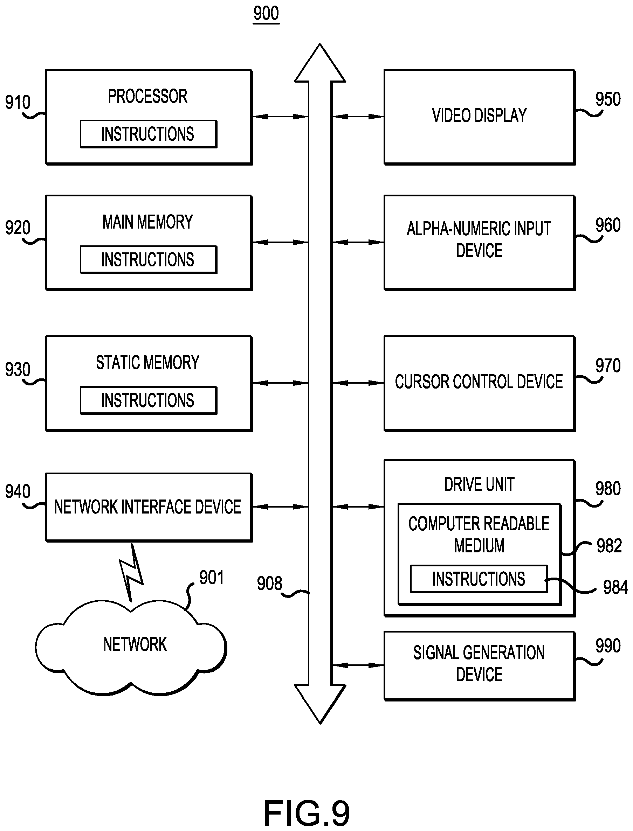

The processes described for FIG. 8 may be performed using instructions stored on a computer readable medium in the console 290 as an example. For example, a processor 292 may execute instructions stored in the memory 291 in order to implement the processes for a console 290 in the embodiment of FIG. 2, though a controller with such a processor 292 and a memory 291 may be implemented in any of the other consoles in other embodiments described herein. An example of a console 290 that can be used to implement the processes of FIG. 8 is described more fully below with respect to FIG. 9.

FIG. 9 illustrates a general computer system, on which a method of encoded synchronized medical intervention image signals and sensor signals can be implemented, in accordance with another representative embodiment.

The computer system 900 can include a set of instructions that can be executed to cause the computer system 900 to perform any one or more of the methods or computer-based functions disclosed herein. The computer system 900 may operate as a standalone device or may be connected, for example, using a network 901, to other computer systems or peripheral devices.

In a networked deployment, the computer system 900 may operate in the capacity of a server or as a client user computer in a server-client user network environment, or as a peer computer system in a peer-to-peer (or distributed) network environment. The computer system 900 can also be implemented as or incorporated into various devices, such as the console 190, the console 290, the console 390, a stationary computer, a mobile computer, a personal computer (PC), a laptop computer, a tablet computer, or any other machine capable of executing a set of instructions (sequential or otherwise) that specify actions to be taken by that machine. The computer system 900 can be incorporated as or in a device that in turn is in an integrated system that includes additional devices. In an embodiment, the computer system 900 can be implemented using electronic devices that provide voice, video or data communication. Further, while the computer system 900 is illustrated in the singular, the term "system" shall also be taken to include any collection of systems or sub-systems that individually or jointly execute a set, or multiple sets, of instructions to perform one or more computer functions.

As illustrated in Fig. 9, the computer system 900 includes a processor 910. A processor for a computer system 900 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. A processor is an article of manufacture and/or a machine component. A processor for a computer system 900 is configured to execute software instructions to perform functions as described in the various embodiments herein. A processor for a computer system 900 may be a general-purpose processor or may be part of an application specific integrated circuit (ASIC). A processor for a computer system 900 may also be a microprocessor, a microcomputer, a processor chip, a controller, a microcontroller, a digital signal processor (DSP), a state machine, or a programmable logic device. A processor for a computer system 900 may also be a logical circuit, including a programmable gate array (PGA) such as a field programmable gate array (FPGA), or another type of circuit that includes discrete gate and/or transistor logic. A processor for a computer system 900 may be a central processing unit (CPU), a graphics processing unit (GPU), or both. Additionally, any processor described herein may include multiple processors, parallel processors, or both. Multiple processors may be included in, or coupled to, a single device or multiple devices.

A "processor" as used herein encompasses an electronic component which is able to execute a program or machine executable instruction. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each including a processor or processors. Many programs have instructions performed by multiple processors that may be within the same computing device or which may even be distributed across multiple computing devices.

Moreover, the computer system 900 may include a main memory 920 and a static memory 930, where memories in the computer system 900 may communicate with each other via a bus 908. Memories described herein are tangible storage mediums that can store data and executable instructions and are non-transitory during the time instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. A memory described herein is an article of manufacture and/or machine component. Memories described herein are computer-readable mediums from which data and executable instructions can be read by a computer. Memories as described herein may be random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. Memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 900 may further include a video display unit 950, such as a liquid crystal display (LCD), an organic light emitting diode (OLED), a flat panel display, a solid-state display, or a cathode ray tube (CRT). Additionally, the computer system 900 may include an input device 960, such as a keyboard/virtual keyboard or touch-sensitive input screen or speech input with speech recognition, and a cursor control device 970, such as a mouse or touch-sensitive input screen or pad. The computer system 900 can also include a disk drive unit 980, a signal generation device 990, such as a speaker or remote control, and a network interface device 940.

In an embodiment, as depicted in Fig. 9, the disk drive unit 980 may include a computer-readable medium 982 in which one or more sets of instructions 984, e.g. software, can be embedded. Sets of instructions 984 can be read from the computer-readable medium 982. Further, the instructions 984, when executed by a processor, can be used to perform one or more of the methods and processes as described herein. In an embodiment, the instructions 984 may reside completely, or at least partially, within the main memory 920, the static memory 930, and/or within the processor 910 during execution by the computer system 900.

In an alternative embodiment, dedicated hardware implementations, such as application-specific integrated circuits (ASICs), programmable logic arrays and other hardware components, can be constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing can be constructed to implement one or more of the methods or functionalities as described herein, and a processor described herein may be used to support a virtual processing environment.

The present disclosure contemplates a computer-readable medium 982 that includes instructions 984 or receives and executes instructions 984 responsive to a propagated signal; so that a device connected to a network 901 can communicate voice, video or data over the network 901. Further, the instructions 984 may be transmitted or received over the network 901 via the network interface device 940.

Accordingly, encoded synchronized medical intervention image signals and sensor signals enables accurate synchronization of timing information indicating the firing of beams by an ultrasound imaging probe, which preempts latency concerns that could be imposed when latency is introduced in later processing. Although encoded synchronized medical intervention image signals and sensor signals has been described with reference to several exemplary embodiments, it is understood that the words that have been used are words of description and illustration, rather than words of limitation.

For example, as explained above the amplification by amplifiers is generally explained before the adding described herein. However, the adding may be performed first to produce a first combined signal and/or a second combined signal that is then amplified subsequently. Additionally, an analog-to-digital converter is generally not shown for converting a signal stream from the ultrasound imaging probes described herein; however, such an analog-to-digital converter may be present and used in any of the module 120, the module 220 or the module 320.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

## Claims

1. A controller (120/220/320) for synchronizing image signals and sensor signals in a medical intervention, comprising:
a circuit (121-126/221-226/321-351) that implements a process comprising:
receiving (S410) a signal stream between an ultrasound imaging probe (110/210/310) that emits a plurality of beams during the medical intervention and a console (190/290/390) that receives the image signals from the ultrasound imaging probe (110/210/310) generated based on the plurality of beams, the signal stream including synchronization information indicating timing of emission of each beam of the plurality of beams;
extracting (S420), by the circuit (121-126/221-226/321-351) from the signal stream, the synchronization information indicating the timing of emission of each beam of the plurality of beams;
receiving (S430), by the circuit (121-126/221-226/321-351) from a first passive ultrasound sensor (S 1) that receives energy from each beam emitted by the ultrasound imaging probe (110/210/310), a first signal that includes first sensor information indicative of a location of the first passive ultrasound sensor (S1) and generated based on receipt by the first passive ultrasound sensor (S1) of the energy received from each beam emitted by the ultrasound imaging probe (110/210/310);
adding (S450) to the first signal with the first sensor information, and based on the synchronization information extracted from the signal stream, a second signal with a predefined signature characteristic indicating the timing of emission of each beam of the plurality of beams, to produce a first combined signal; and
sending (S460), from the circuit (121-126/221-226/321-351) to the console (190/290/390), the first combined signal produced by adding the first signal with the first sensor information and the second signal with the predefined signature characteristic indicating the timing of emission of each beam of the plurality of beams.

2. The controller (120/320) of claim 1,
wherein the signal stream is received from the ultrasound imaging probe (110/310) and includes images from the ultrasound imaging probe (110/310) among the image signals for the console (190/390), and
the console (190//390) generates (S480) a display of the images and the location of the first passive ultrasound sensor (S1) synchronized based on the predefined signature characteristic.

3. The controller (120/220/320) of claim 1,
wherein the adding comprises combining the first signal with a predefined waveform as the predefined signature characteristic so that the console (190/290/390) can detect the predefined waveform in the first combined signal.

4. The controller (120/220/320) of claim 1,
wherein the adding comprises combining the first signal with a first predefined waveform and a second predefined waveform as the predefined signature characteristic so that the console (190/290/390) can detect the first predefined waveform and the second predefined waveform in the first combined signal,
the first predefined waveform corresponds to a frame trigger, and
the second predefined waveform corresponds to a line trigger.

5. The controller (120/220/320) of claim 1,
wherein the adding comprises combining the first signal with a positive voltage pulse as the predefined signature characteristic so that the console (190/290/390) can detect the positive voltage pulse in the first combined signal.

6. The controller (220) of claim 1,
wherein the signal stream is received from the console (290), and
the console (290) generates a display of images from the image signals from the ultrasound imaging probe (210) and the location of the first passive ultrasound sensor (S1) synchronized based on the predefined signature characteristic.

7. The controller (320) of claim 1, wherein the process implemented by the circuit (321-351) further comprises:
amplifying (S540), by the circuit (321-351), an output of the first passive ultrasound sensor (S1) to produce an amplified first signal;
digitizing (S545), by the circuit (321-351), the amplified first signal to produce the first signal, wherein the adding comprises combining the first signal with at least one pulse representing at least one digital bit as the predefined signature characteristic, and
transmitting (S560), by the circuit (321-351), the first combined signal.

8. The controller (320) of claim 1, wherein the process implemented by the circuit (321-351) further comprises:
receiving (S630) an output from the first passive ultrasound sensor (S1) as a first sensor output;
digitizing (S645), by the circuit (321-351), the first sensor output to produce a digitized sensor output;
digitizing (S647), by the circuit (121-126/221-226/321-351), the signal stream to produce a digitized signal stream, and
combining (S650) the digitized signal stream and the digitized sensor output to produce a digitized first combined signal as the first combined signal.

9. The controller (320) of claim 8, further comprising:
transmitting, by the circuit (321-351), the digitized first combined signal for receipt by a receiver (352) that interfaces with the console (390).

10. The controller (120/220/320) of claim 1, wherein the process implemented by the circuit (121-126/221-226/321-351) further comprises:
receiving (S753), by the circuit (121-126/221-226/321-351) from a second passive ultrasound sensor (S2) that receives energy from each beam emitted by the ultrasound imaging probe (110/210/310), a third signal that includes second sensor information indicative of a location of the second passive ultrasound sensor (S2) and generated based on receipt by the second passive ultrasound sensor (S2) of the energy received from each beam emitted by the ultrasound imaging probe (110/210/310);
adding (S757) to the third signal with the second sensor information, and based on the synchronization information extracted from the signal stream, the second signal with the predefined signature characteristic indicating the timing of emission of each beam of the plurality of beams, to produce a second combined signal; and
sending (S760), from the circuit (121-126/221-226/321-351) to the console, the second combined signal produced by adding the third signal with the second sensor information and the second signal with the predefined signature characteristic indicating the timing of emission of each beam of the plurality of beams.

11. A system (100/200/300) for synchronizing image signals and sensor signals in a medical intervention, comprising:
an ultrasound imaging probe (110/210/310) that emits a plurality of beams during the medical intervention;
a console (190/290/390) that receives image signals from the ultrasound imaging probe (110/210/310) generated based on the plurality of beams;
a first passive ultrasound sensor (S1) that receives energy from each beam emitted by the ultrasound imaging probe (110/210/310), and
a controller (120/220/320) with a circuit (121-126/221-226/321-351) that implements a process comprising:
receiving (S410) a signal stream between the ultrasound imaging probe (110/210/310) and the console, the signal stream including synchronization information indicating timing of emission of each beam of the plurality of beams;
extracting (S420), by the circuit (121-126/221-226/321-351) from the signal stream, the synchronization information indicating the timing of emission of each beam of the plurality of beams;
receiving (S430), by the circuit (121-126/221-226/321-351) from the first passive ultrasound sensor, a first signal that includes first sensor information indicative of a location of the first passive ultrasound sensor (S1) and generated based on receipt by the first passive ultrasound sensor (S1) of the energy received from each beam emitted by the ultrasound imaging probe (110/210/310);
adding (S450) to the first signal with the first sensor information, and based on the synchronization information extracted from the signal stream, a second signal with a predefined signature characteristic indicating the timing of emission of each beam of the plurality of beams, to produce a combined signal; and
sending (S460), from the circuit (121-126/221-226/321-351) to the console, the combined signal produced by adding the first signal with the first sensor information and the second signal with the predefined signature characteristic indicating the timing of emission of each beam of the plurality of beams.

12. The system (100/200/300) of claim 11, further comprising:
a second passive ultrasound sensor (S2) that receives energy from each beam emitted by the ultrasound imaging probe (110/210/310),
wherein the process implemented by the circuit (121-126/221-226/321-351) further comprises:
receiving (S753), by the circuit (121-126/221-226/321-351) from the second passive ultrasound sensor (S2), a third signal that includes second sensor information indicative of a location of the second passive ultrasound sensor (S2) and generated based on receipt by the second passive ultrasound sensor (S2) of the energy received from each beam emitted by the ultrasound imaging probe (110/210/310);
adding (757) to the third signal with the second sensor information, and based on the synchronization information extracted from the signal stream, the second signal with the predefined signature characteristic indicating the timing of emission of each beam of the plurality of beams, to produce a second combined signal; and
sending (S760), from the circuit (121-126/221-226/321-351) to the console (190/290/390), the second combined signal produced by adding the third signal with the second sensor information and the second signal with the predefined signature characteristic indicating the timing of emission of each beam of the plurality of beams.

13. The system (100/200/300) of claim 11,
wherein the adding comprises combining the first signal with a predefined waveform as the predefined signature characteristic so that the console (190/290/390) can detect the predefined waveform in the combined signal.

14. The system (100/200/300) of claim 11,
wherein the adding comprises combining the first signal with a first predefined waveform and a second predefined waveform as the predefined signature characteristic so that the console (190/290/390) can detect the first predefined waveform and the second predefined waveform in the combined signal,
the first predefined waveform corresponds to a frame trigger, and
the second predefined waveform corresponds to a line trigger.

15. The system (100/200/300) of claim 11,
wherein the adding comprises combining the first signal with a positive voltage pulse as the predefined signature characteristic so that the console (190/290/390) can detect the positive voltage pulse in the combined signal.

16. A computer program comprising computer readable code or instructions, which, when executed by a processor (292), causes a console (290) to perform a process for synchronizing image signals and sensor signals in a medical intervention, the computer program comprising:
receiving (S810), from an ultrasound imaging probe (210), image signals generated based on a plurality of beams emitted by the ultrasound imaging probe (210);
receiving (S870) a first combined signal produced by adding a first signal with first sensor information and a second signal with a predefined signature characteristic indicating timing emission of each beam of the plurality of beams, wherein the first sensor information is indicative of a location of a first passive ultrasound sensor (S 1) and is generated based on receipt of energy from each beam emitted by the ultrasound imaging probe (210) by the first passive ultrasound sensor (S 1);
separating (S872) the first signal with the first sensor information from the second signal with the predefined signature characteristic;
obtaining (S874), from the second signal, synchronization information indicating timing of emission of each beam of the plurality of beams, and
synchronizing (S876), based on the synchronization information obtained from the second signal, images from the ultrasound imaging probe (210) with sensor data of the first passive ultrasound sensor (S1) obtained from the first sensor information of the first signal.

17. A tangible non-transitory computer readable storage medium (291) that stores a computer program as claimed in claim 16,

18. The tangible non-transitory computer readable storage medium (291) of claim 16, wherein, when executed by the processor (292), the computer program further causes the console (290) to generate (S880) a display of the images from the ultrasound imaging probe (210) synchronized with locations of the first passive ultrasound sensor (S 1).

19. The tangible non-transitory computer readable storage medium (291) of claim 16, wherein the second signal comprises a predefined waveform, and
when executed by the processor (292), the computer program further causes the console (290) to detect the predefined waveform in the first combined signal.

20. The tangible non-transitory computer readable storage medium (291) of claim 16, wherein the second signal comprises a first predefined waveform and a second predefined waveform,
when executed by the processor (292), the computer program further causes the console (190/290/390) to detect the first predefined waveform and the second predefined waveform in the first combined signal,
the first predefined waveform corresponds to a frame trigger, and
the second predefined waveform corresponds to a line trigger.

## Patentansprüche

1. Steuerung (120/220/320) zum Synchronisieren von Bildsignalen und Sensorsignalen bei einem medizinischen Eingriff, umfassend:
eine Schaltung (121-126/221-226/321-351), die einen Prozess implementiert, umfassend:
Empfangen (S410) eines Signalstroms zwischen einer Ultraschallbildgebungssonde (110/210/310), die während des medizinischen Eingriffs eine Vielzahl von Strahlen emittiert, und einer Konsole (190/290/390), die die Bildsignale von der Ultraschallbildgebungssonde (110/210/310) empfängt, die basierend auf der Vielzahl von Strahlen erzeugt werden, wobei der Signalstrom Synchronisationsinformationen beinhaltet, die einen Emissionszeitpunkt von jedem Strahl der Vielzahl von Strahlen angeben;
Extrahieren (S420), durch die Schaltung (121-126/221-226/321-351) aus dem Signalstrom, der Synchronisationsinformation, die den Emissionszeitpunkt von jedem Strahl der Vielzahl von Strahlen angeben; Empfangen (S430), durch die Schaltung (121-126/221-226/321-351) von einem ersten passiven Ultraschallsensor (S1), der Energie von jedem von der Ultraschallbildgebungssonde (110/210/310) emittierten Strahl empfängt, eines ersten Signals, das erste Sensorinformationen beinhaltet, die indikativ für eine Stelle des ersten passiven Ultraschallsensors (S1) sind und basierend auf einem Empfang, durch den ersten passiven Ultraschallsensor (S1), der Energie, die von jedem Strahl empfangen wird, der von der Ultraschallbildgebungssonde (110/210/310) emittiert wird, erzeugt wird;
Addieren (S450), zu dem ersten Signal mit den ersten Sensorinformationen und basierend auf den aus dem Signalstrom extrahierten Synchronisationsinformationen, eines zweiten Signals mit einer vordefinierten Signaturcharakteristik, die den Emissionszeitpunkt von jedem Strahl der Vielzahl von Strahlen angibt, um ein erstes kombiniertes Signal zu erzeugen; und
Senden (S460), von der Schaltung (121-126/221-226/321-351) an die Konsole (190/290/390), des ersten kombinierten Signals, das durch Addieren des ersten Signals mit den ersten Sensorinformationen und des zweiten Signals mit der vordefinierten Signaturcharakteristik erzeugt wird, die den Emissionszeitpunkt von jedem Strahl der Vielzahl von Strahlen angibt.

2. Steuerung (120/320) nach Anspruch 1,
wobei der Signalstrom von der Ultraschallbildgebungssonde (110/310) empfangen wird und Bilder von der Ultraschallbildgebungssonde (110/310) unter den Bildsignalen für die Konsole (190/390) beinhaltet, und
die Konsole (190//390) eine Anzeige der Bilder und der Stelle des ersten passiven Ultraschallsensors (S1) erzeugt (S480), die basierend auf der vordefinierten Signaturcharakteristik synchronisiert sind.

3. Steuerung (120/220/320) nach Anspruch 1,
wobei das Addieren ein Kombinieren des ersten Signals mit einer vordefinierten Wellenform als die vordefinierte Signaturcharakteristik umfasst, sodass die Konsole (190/290/390) die vordefinierte Wellenform in dem ersten kombinierten Signal erkennen kann.

4. Steuerung (120/220/320) nach Anspruch 1,
wobei das Addieren ein Kombinieren des ersten Signals mit einer ersten vordefinierten Wellenform und einer zweiten vordefinierten Wellenform als die vordefinierte Signaturcharakteristik umfasst, sodass die Konsole (190/290/390) die erste vordefinierte Wellenform und die zweite vordefinierte Wellenform in dem ersten kombinierten Signal erkennen kann,
die erste vordefinierte Wellenform einem Frame-Trigger entspricht, und
die zweite vordefinierte Wellenform einem Line-Trigger entspricht.

5. Steuerung (120/220/320) nach Anspruch 1,
wobei das Addieren ein Kombinieren des ersten Signals mit einem positiven Spannungsimpuls als die vordefinierte Signaturcharakteristik umfasst, sodass die Konsole (190/290/390) den positiven Spannungsimpuls in dem ersten kombinierten Signal erkennen kann.

6. Steuerung (220) nach Anspruch 1,
wobei der Signalstrom von der Konsole (290) empfangen wird, und die Konsole (290) eine Anzeige von Bildern aus den Bildsignalen von der Ultraschallbildgebungssonde (210) und der Stelle des ersten passiven Ultraschallsensors (S1), die basierend auf der vordefinierten Signaturcharakteristik synchronisiert sind.

7. Steuerung (320) nach Anspruch 1, wobei der durch die Schaltung (321-351) implementierte Prozess ferner Folgendes umfasst:
Verstärken (S540), durch die Schaltung (321-351), eines Ausgangs des ersten passiven Ultraschallsensors (S1), um ein verstärktes erstes Signal zu erzeugen; Digitalisieren (S545) des verstärkten ersten Signals durch die Schaltung (321-351), um das erste Signal zu erzeugen, wobei das Addieren ein Kombinieren des ersten Signals mit mindestens einem Impuls umfasst, der mindestens ein digitales Bit als die vordefinierte Signaturcharakteristik darstellt, und Übertragen (S560), durch die Schaltung (321-351), des ersten kombinierten Signals.

8. Steuerung (320) nach Anspruch 1, wobei der durch die Schaltung (321-351) implementierte Prozess ferner Folgendes umfasst:
Empfangen (S630) eines Ausgangs von dem ersten passiven Ultraschallsensor (S1) als ersten Sensorausgang; Digitalisieren (S645), durch die Schaltung (321-351), des ersten Sensorausgangs, um einen digitalisierten Sensorausgang zu erzeugen;
Digitalisieren (S647), durch die Schaltung (121-126/221-226/321-351) des Signalstroms, um einen digitalisierten Signalstrom zu erzeugen, und
Kombinieren (S650) des digitalisierten Signalstroms und des digitalisierten Sensorausgangs, um ein digitalisiertes erstes kombiniertes Signal als das erste kombinierte Signal zu erzeugen.

9. Steuerung (320) nach Anspruch 8, ferner umfassend:
Übertragen, durch die Schaltung (321-351), des digitalisierten ersten kombinierten Signals zum Empfang durch einen Empfänger (352), der mit der Konsole (390) verbunden ist.

10. Steuerung (120/220/320) nach Anspruch 1, wobei der durch die Schaltung (121-126/221-226/321-351) implementierte Prozess ferner Folgendes umfasst:
Empfangen (S753), durch die Schaltung (121-126/221-226/321-351) von einem zweiten passiven Ultraschallsensor (S2), der Energie von jedem von der Ultraschallbildgebungssonde (110/210/310) emittierten Strahl empfängt, eines dritten Signals, das erste Sensorinformationen beinhaltet, die indikativ für eine Stelle des zweiten passiven Ultraschallsensors (S2) sind und basierend auf einem Empfang, durch den zweiten passiven Ultraschallsensor (S2), der Energie, die von jedem Strahl empfangen wird, der von der Ultraschallbildgebungssonde (110/210/310) emittiert wird, erzeugt wird;
Addieren (S757), zu dem dritten Signal mit den zweiten Sensorinformationen und basierend auf den aus dem Signalstrom extrahierten Synchronisationsinformationen, des zweiten Signals mit der vordefinierten Signaturcharakteristik, die den Emissionszeitpunkt von jedem Strahl der Vielzahl von Strahlen angibt, um ein zweites kombiniertes Signal zu erzeugen; und
Senden (S760), von der Schaltung (121-126/221-226/321-351) an die Konsole, des zweiten kombinierten Signals, das durch Addieren des dritten Signals mit den zweiten Sensorinformationen und des zweiten Signals mit der vordefinierten Signaturcharakteristik erzeugt wird, die den Emissionszeitpunkt von jedem Strahl der Vielzahl von Strahlen angibt.

11. System (100/200/300) zum Synchronisieren von Bildsignalen und Sensorsignalen bei einem medizinischen Eingriff, umfassend:
eine Ultraschallbildgebungssonde (110/210/310), die während des medizinischen Eingriffs eine Vielzahl von Strahlen emittiert;
eine Konsole (190/290/390), die Bildsignale von der Ultraschallbildgebungssonde (110/210/310) empfängt, die basierend auf der Vielzahl von Strahlen erzeugt werden; einen ersten passiven Ultraschallsensor (S1), der Energie von jedem Strahl empfängt, der von der Ultraschallbildgebungssonde (110/210/310) emittiert wird, und eine Steuerung (120/220/320) mit einer Schaltung (121-126/221-226/321-351), die einen Prozess implementiert, umfassend:
Empfangen (S410) eines Signalstroms zwischen der Ultraschallbildgebungssonde (110/210/310) und der Konsole, wobei der Signalstrom Synchronisationsinformationen beinhaltet, die den Emissionszeitpunkt von jedem Strahl der Vielzahl von Strahlen angeben;
Extrahieren (S420), durch die Schaltung (121-126/221-226/321-351) aus dem Signalstrom, der Synchronisationsinformation, die den Emissionszeitpunkt von jedem Strahl der Vielzahl von Strahlen angeben; Empfangen (S430), durch die Schaltung (121-126/221-226/321-351) von dem ersten passiven Ultraschallsensor, eines ersten Signals, das erste Sensorinformationen beinhaltet, die eine Stelle des ersten passiven Ultraschallsensors (S1) angeben, und das basierend auf einem Empfang, durch den ersten passiven Ultraschallsensor (S1), der Energie erzeugt wird, die von jedem Strahl empfangen wird, der von der Ultraschallbildgebungssonde (110/210/310) emittiert wird; Addieren (S450), zu dem Signal mit den ersten Sensorinformationen und basierend auf den aus dem Signalstrom extrahierten Synchronisationsinformationen, eines zweiten Signals mit einer vordefinierten Signaturcharakteristik, die den Emissionszeitpunkt von jedem Strahl der Vielzahl von Strahlen angibt, um ein kombiniertes Signal zu erzeugen; und
Senden (S460), von der Schaltung (121-126/221-226/321-351) an die Konsole, des kombinierten Signals, das durch Addieren des ersten Signals mit den ersten Sensorinformationen und des zweiten Signals mit der vordefinierten Signaturcharakteristik erzeugt wird, die den Emissionszeitpunkt von jedem Strahl der Vielzahl von Strahlen angibt.

12. System (100/200/300) nach Anspruch 11, ferner umfassend:
einen zweiten passiven Ultraschallsensor (S2), der die Energie von jedem Strahl empfängt, der von der bildgebenden Ultraschallsonde (110/210/310) emittiert wird, wobei der von der Schaltung (121-126/221-226/321-351) implementierte Prozess ferner Folgendes umfasst: Empfangen (S753), durch die Schaltung (121-126/221-226/321-351) von dem zweiten passiven Ultraschallsensor (S2) eines dritten Signals, das erste Sensorinformationen beinhaltet, die indikativ für eine Stelle des zweiten passiven Ultraschallsensors (S2) sind und basierend auf einem Empfang, durch den zweiten passiven Ultraschallsensor (S2), der Energie, die von jedem Strahl empfangen wird, der von der Ultraschallbildgebungssonde (110/210/310) emittiert wird, erzeugt wird;
Addieren (757), zu dem dritten Signal mit den zweiten Sensorinformationen und basierend auf den aus dem Signalstrom extrahierten Synchronisationsinformationen, des zweiten Signals mit der vordefinierten Signaturcharakteristik, die den Emissionszeitpunkt von jedem Strahl der Vielzahl von Strahlen angibt, um ein zweites kombiniertes Signal zu erzeugen; und
Senden (S760), von der Schaltung (121-126/221-226/321-351) an die Konsole (190/290/390), des zweiten kombinierten Signals, das durch Addieren des dritten Signals mit den zweiten Sensorinformationen und des zweiten Signals mit der vordefinierten Signaturcharakteristik erzeugt wird, die den Emissionszeitpunkt von jedem Strahl der Vielzahl von Strahlen angibt.

13. System (100/200/300) nach Anspruch 11,
wobei das Addieren ein Kombinieren des ersten Signals mit einer vordefinierten Wellenform als die vordefinierte Signaturcharakteristik umfasst, sodass die Konsole (190/290/390) die vordefinierte Wellenform in dem kombinierten Signal erkennen kann.

14. System (100/200/300) nach Anspruch 11,
wobei das Addieren ein Kombinieren des Signals mit einer ersten vordefinierten Wellenform und einer zweiten vordefinierten Wellenform als die vordefinierte Signaturcharakteristik umfasst, sodass die Konsole (190/290/390) die erste vordefinierte Wellenform und die zweite vordefinierte Wellenform in dem kombinierten Signal erkennen kann,
die erste vordefinierte Wellenform einem Frame-Trigger entspricht und
die zweite vordefinierte Wellenform einem Line-Trigger entspricht.

15. System (100/200/300) nach Anspruch 11,
wobei das Addieren ein Kombinieren des ersten Signals mit einem positiven Spannungsimpuls als die vordefinierte Signaturcharakteristik umfasst, sodass die Konsole (190/290/390) den positiven Spannungsimpuls in dem kombinierten Signal erkennen kann.

16. Computerprogramm, umfassend computerlesbaren Code oder Anweisungen, die, wenn sie von einem Prozessor (292) ausgeführt werden, eine Konsole (290) veranlasst, einen Prozess zum Synchronisieren von Bildsignalen und Sensorsignalen bei einem medizinischen Eingriff durchzuführen, das Computerprogramm umfassend:
Empfangen (S810), von einer Ultraschallbildgebungssonde (210), von Bildsignalen, die basierend auf einer Vielzahl von Strahlen erzeugt werden, die von der Ultraschallbildgebungssonde (210) emittiert werden; Empfangen (S870) eines ersten kombinierten Signals, das durch Addieren eines ersten Signals mit ersten Sensorinformationen und eines zweiten Signals mit einer vordefinierten Signaturcharakteristik erzeugt wird, die den Emissionszeitpunkt von jedem Strahl der Vielzahl von Strahlen angibt, wobei die ersten Sensorinformationen eine Stelle eines ersten passiven Ultraschallsensors (S1) angeben und basierend auf einem Empfang von Energie von jedem von der Ultraschallbildgebungssonde (210) emittierten Strahl durch den ersten passiven Ultraschallsensor (S1) erzeugt werden;
Trennen (S872) des ersten Signals mit den ersten Sensorinformationen von dem zweiten Signal mit der vordefinierten Signaturcharakteristik;
Erlangen (S874) von Synchronisationsinformationen aus dem zweiten Signal, die den Emissionszeitpunkt von jedem Strahl der Vielzahl von Strahlen angeben, und Synchronisieren (S876), basierend auf den aus dem zweiten Signal erlangten Synchronisationsinformationen, von Bildern der Ultraschallbildgebungssonde (210) mit Sensordaten des ersten passiven Ultraschallsensors (S1), die aus den ersten Sensorinformationen des ersten Signals erlangt werden.

17. Greifbares, nicht-übertragbares, computerlesbares Speichermedium (291), das ein Computerprogramm nach Anspruch 16 speichert.

18. Greifbares, nicht-übertragbares, computerlesbares Speichermedium (291) nach Anspruch 16 wobei das Computerprogramm, wenn es von dem Prozessor (292) ausgeführt wird, ferner die Konsole (290) veranlasst, eine Anzeige der Bilder von der Ultraschallbildgebungssonde (210) zu erzeugen (S880), die mit Stellen des ersten passiven Ultraschallsensors (S1) synchronisiert ist.

19. Greifbares, nicht-übertragbares, computerlesbares Speichermedium (291) nach Anspruch 16, wobei das zweite Signal eine vordefinierte Wellenform umfasst, und
das Computerprogramm, wenn es vom Prozessor (292) ausgeführt wird, die Konsole (290) ferner veranlasst, die vordefinierte Wellenform in dem ersten kombinierten Signal zu erkennen.

20. Greifbares, nicht-übertragbares, computerlesbares Speichermedium (291) nach Anspruch 16, wobei das zweite Signal eine erste vordefinierte Wellenform und eine zweite vordefinierte Wellenform umfasst, wobei das Computerprogramm,
wenn es von dem Prozessor (292) ausgeführt wird, ferner bewirkt, dass die Konsole (190/290/390) die erste vordefinierte Wellenform und die zweite vordefinierte Wellenform in dem ersten kombinierten Signal erkennt, die erste vordefinierte Wellenform einem Frame-Trigger entspricht, und die zweite vordefinierte Wellenform einem Line-Trigger entspricht.

## Revendications

1. Contrôleur (120/220/320) pour la synchronisation des signaux d'image et des signaux de capteur dans le cadre d'une intervention médicale, comprenant:
un circuit (121-126/221-226/321-351) qui met en oeuvre un processus comprenant les étapes consistant à:
recevoir (S410) un flux de signaux entre une sonde d'imagerie par ultrasons (110/210/310) qui émet une pluralité de faisceaux pendant l'intervention médicale et une console (190/290/390) qui reçoit les signaux d'image de la sonde d'imagerie par ultrasons (110/210/310) générés sur la base de la pluralité de faisceaux, le flux de signaux comprenant des informations de synchronisation indiquant le moment de l'émission de chaque faisceau de la pluralité de faisceaux;
extraire (S420), par le circuit (121-126/221-226/321-351) du flux de signaux, des informations de synchronisation indiquant le moment de l'émission de chaque faisceau de la pluralité de faisceaux;
recevoir (S430), par le circuit (121-126/221-226/321-351) d'un premier capteur passif à ultrasons (S1) qui reçoit l'énergie de chaque faisceau émis par la sonde d'imagerie par ultrasons (110/210/310), un premier signal qui comprend les premières informations du capteur indiquant un emplacement du premier capteur passif à ultrasons (S1) et généré sur la base de la réception par le premier capteur passif à ultrasons (S1) de l'énergie reçue de chaque faisceau émis par la sonde d'imagerie par ultrasons (110/210/310);
ajouter (S450) au premier signal avec les premières informations du capteur, et sur la base des informations de synchronisation extraites du flux de signaux, un second signal avec une caractéristique de signature prédéfinie indiquant le moment de l'émission de chaque faisceau de la pluralité de faisceaux, afin de produire un premier signal combiné; et
envoyer (S460), du circuit (121-126/221-226/321-351) à la console (190/290/390), le premier signal combiné produit par l'addition du premier signal avec les premières informations du capteur et du second signal avec la caractéristique de signature prédéfinie indiquant le moment de l'émission de chaque faisceau de la pluralité de faisceaux.

2. Contrôleur (120/320) selon la revendication 1,
dans lequel le flux de signaux est reçu de la sonde d'imagerie par ultrasons (110/310) et comprend des images de la sonde d'imagerie par ultrasons (110/310) parmi les signaux d'image pour la console (190/390), et la console (190/390) génère (S480) un affichage des images et de l'emplacement du premier capteur passif à ultrasons (S1) synchronisé sur la base de la caractéristique de signature prédéfinie.

3. Contrôleur (120/220/320) selon la revendication 1, dans lequel l'addition comprend la combinaison du premier signal avec une forme d'onde prédéfinie en tant que caractéristique de signature prédéfinie de sorte que la console (190/290/390) puisse détecter la forme d'onde prédéfinie dans le premier signal combiné.

4. Contrôleur (120/220/320) selon la revendication 1, dans lequel l'addition comprend la combinaison du premier signal avec une première forme d'onde prédéfinie et une seconde forme d'onde prédéfinie comme caractéristique de signature prédéfinie de sorte que la console (190/290/390) puisse détecter la première forme d'onde prédéfinie et la seconde forme d'onde prédéfinie dans le premier signal combiné, la première forme d'onde prédéfinie correspond à un déclencheur de trame, et la seconde forme d'onde prédéfinie correspond à un déclencheur de ligne.

5. Contrôleur (120/220/320) selon la revendication 1, dans lequel l'addition comprend la combinaison du premier signal avec une impulsion de tension positive en tant que caractéristique de signature prédéfinie de sorte que la console (190/290/390) puisse détecter l'impulsion de tension positive dans le premier signal combiné.

6. Contrôleur (220) selon la revendication 1,
dans lequel le flux de signaux est reçu de la console (290), et la console (290) génère un affichage d'images à partir des signaux d'image provenant de la sonde d'imagerie par ultrasons (210) et de l'emplacement du premier capteur passif à ultrasons (S1) synchronisé sur la base de la caractéristique de signature prédéfinie.

7. Contrôleur (320) selon la revendication 1,
dans lequel le processus mis en oeuvre par le circuit (321-351) comprend en outre les étapes consistant à:
l'amplification (S540), par le circuit (321-351), d'une sortie du premier capteur passif à ultrasons (S1) pour produire un premier signal amplifié;
la numérisation (S545), par le circuit (321-351), du premier signal amplifié pour produire le premier signal, l'addition consistant à combiner le premier signal avec au moins une impulsion représentant au moins un bit numérique en tant que caractéristique de signature prédéfinie, et la transmission (S560), par le circuit (321-351), du premier signal combiné.

8. Contrôleur (320) selon la revendication 1, dans lequel le processus mis en oeuvre par le circuit (321-351) comprend en outre:
recevoir (S630) une sortie du premier capteur passif à ultrasons (S1) en tant que première sortie de capteur; numériser (S645), par le circuit (321-351), la première sortie de capteur pour produire une sortie de capteur numérisée;
numériser (S647), par le circuit (121-126/221-226/321-351), le flux de signaux pour produire un flux de signaux numérisés, et combiner (S650) le flux de signaux numérisés et la sortie numérisée du capteur pour produire un premier signal combiné numérisé en tant que premier signal combiné.

9. Contrôleur (320) selon la revendication 8, comprenant en outre:
la transmission, par le circuit (321-351), du premier signal combiné numérisé pour réception par un récepteur (352) en interface avec la console (390).

10. Contrôleur (120/220/320) selon la revendication 1, dans lequel le processus mis en oeuvre par le circuit (121-126/221-226/321-351) comprend en outre les étapes consistant à:
recevoir (S753), par le circuit (121-126/221-226/321-351) d'un second capteur passif à ultrasons (S2) qui reçoit l'énergie de chaque faisceau émis par la sonde d'imagerie par ultrasons (110/210/310), un troisième signal comprenant des informations sur le second capteur indiquant l'emplacement du second capteur passif à ultrasons (S2) et généré sur la base de la réception par le second capteur passif à ultrasons (S2) de l'énergie reçue de chaque faisceau émis par la sonde d'imagerie par ultrasons (110/210/310);
ajouter (S757) au troisième signal avec les secondes informations du capteur, et sur la base des informations de synchronisation extraites du flux de signaux, le second signal avec la caractéristique de signature prédéfinie indiquant le moment de l'émission de chaque faisceau de la pluralité de faisceaux, afin de produire un second signal combiné; et
envoyer (S760), du circuit (121-126/221-226/321-351) à la console, le second signal combiné produit par l'addition du troisième signal avec la deuxième information du capteur et le second signal avec la caractéristique de signature prédéfinie indiquant le moment de l'émission de chaque faisceau de la pluralité de faisceaux.

11. A Système (100/200/300) de synchronisation des signaux d'images et des signaux de capteurs dans le cadre d'une intervention médicale, comprenant:
une sonde d'imagerie par ultrasons (110/210/310) qui émet plusieurs faisceaux pendant l'intervention médicale ;
une console (190/290/390) qui reçoit les signaux d'image de la sonde d'imagerie par ultrasons (110/210/310) générés sur la base de la pluralité de faisceaux;
un premier capteur passif à ultrasons (S1) qui reçoit l'énergie de chaque faisceau émis par la sonde d'imagerie par ultrasons (110/210/310), et
un contrôleur (120/220/320) avec un circuit (121-126/221-226/321-351) qui met en oeuvre un processus comprenant:
recevoir (S410) un flux de signaux entre la sonde d'imagerie par ultrasons (110/210/310) et la console, le flux de signaux comprenant des informations de synchronisation indiquant le moment de l'émission de chaque faisceau de la pluralité de faisceaux;
extraire (S420), par le circuit (121-126/221-226/321-351) du flux de signaux, des informations de synchronisation indiquant le moment de l'émission de chaque faisceau de la pluralité de faisceaux;
recevoir (S430), par le circuit (121-126/221-226/321-351) du premier capteur à ultrasons passif, un premier signal comprenant des informations sur le premier capteur indiquant l'emplacement du premier capteur à ultrasons passif (S1) et généré sur la base de la réception par le premier capteur à ultrasons passif (S1) de l'énergie reçue de chaque faisceau émis par la sonde d'imagerie par ultrasons (110/210/310);
ajouter (S450) au premier signal avec les premières informations du capteur, et sur la base des informations de synchronisation extraites du flux de signaux, un second signal avec une caractéristique de signature prédéfinie indiquant le moment de l'émission de chaque faisceau de la pluralité de faisceaux, pour produire un signal combiné; et
envoyer (S460), du circuit (121-126/221-226/321-351) à la console, le signal combiné produit par l'addition du premier signal avec les premières informations du capteur et du second signal avec la caractéristique de signature prédéfinie indiquant le moment de l'émission de chaque faisceau de la pluralité de faisceaux.

12. Système (100/200/300) selon la revendication 11, comprenant en outre:
un second capteur passif à ultrasons (S2) qui reçoit l'énergie de chaque faisceau émis par la sonde d'imagerie par ultrasons (110/210/310),
dans lequel le processus mis en oeuvre par le circuit (121-126/221-226/321-351) comprend en outre:
recevoir (S753), par le circuit (121-126/221-226/321-351) du second capteur passif à ultrasons (S2), un troisième signal comprenant des informations sur le second capteur indiquant l'emplacement du second capteur passif à ultrasons (S2) et généré sur la base de la réception par le second capteur passif à ultrasons (S2) de l'énergie reçue de chaque faisceau émis par la sonde d'imagerie par ultrasons (110/210/310) ;
ajouter (757) au troisième signal avec les informations du second capteur, et sur la base des informations de synchronisation extraites du flux de signaux, le second signal avec la caractéristique de signature prédéfinie indiquant le moment de l'émission de chaque faisceau de la pluralité de faisceaux, pour produire un second signal combiné; et
envoyer (S760), du circuit (121-126/221-226/321-351) à la console (190/290/390), le second signal combiné produit en ajoutant le troisième signal avec les informations du second capteur et le second signal avec la caractéristique de signature prédéfinie indiquant le moment de l'émission de chaque faisceau de la pluralité de faisceaux.

13. Système (100/200/300) selon la revendication 11, dans lequel l'addition comprend la combinaison du premier signal avec une forme d'onde prédéfinie comme caractéristique de signature prédéfinie de sorte que la console (190/290/390) puisse détecter la forme d'onde prédéfinie dans le signal combiné.

14. Système (100/200/300) selon la revendication 11, dans lequel l'addition comprend la combinaison du premier signal avec une première forme d'onde prédéfinie et une seconde forme d'onde prédéfinie comme caractéristique de signature prédéfinie de sorte que la console (190/290/390) puisse détecter la première forme d'onde prédéfinie et la seconde forme d'onde prédéfinie dans le signal combiné,
la première forme d'onde prédéfinie correspond à un déclencheur de trame et la seconde forme d'onde prédéfinie correspond à un déclencheur de ligne.

15. Système (100/200/300) selon la revendication 11, dans lequel l'addition comprend la combinaison du premier signal avec une impulsion de tension positive comme caractéristique de signature prédéfinie de sorte que la console (190/290/390) puisse détecter l'impulsion de tension positive dans le signal combiné.

16. Programme informatique comprenant un code ou des instructions lisibles par ordinateur qui, lorsqu'il est exécuté par un processeur (292), amène une console (290) à effectuer un processus de synchronisation des signaux d'image et des signaux de capteur dans une intervention médicale, le programme informatique comprenant:
recevoir (S810), à partir d'une sonde d'imagerie par ultrasons (210), de signaux d'image générés sur la base d'une pluralité de faisceaux émis par la sonde d'imagerie par ultrasons (210);
recevoir (S870) un premier signal combiné produit par l'addition d'un premier signal avec les premières informations du capteur et d'un second signal avec une caractéristique de signature prédéfinie indiquant la synchronisation de l'émission de chaque faisceau de la pluralité de faisceaux, les premières informations du capteur indiquant l'emplacement d'un premier capteur passif à ultrasons (S1) et étant générées sur la base de la réception de l'énergie de chaque faisceau émis par la sonde d'imagerie par ultrasons (210) par le premier capteur passif à ultrasons (S1);
séparer (S872) le premier signal avec les premières informations du capteur du second signal avec la caractéristique de signature prédéfinie;
obtenir (S874), à partir du second signal, des informations de synchronisation indiquant le moment de l'émission de chaque faisceau de la pluralité de faisceaux, et synchroniser (S876), sur la base des informations de synchronisation obtenues à partir du second signal, les images de la sonde d'imagerie par ultrasons (210) avec les données du capteur du premier capteur passif à ultrasons (S1) obtenues à partir des premières informations de capteur du premier signal.

17. Support de stockage tangible non transitoire lisible par ordinateur (291) qui stocke un programme d'ordinateur tel que revendiqué dans la revendication 16.

18. Support de stockage tangible non transitoire lisible par ordinateur (291) selon la revendication 16 dans lequel, lorsqu'il est exécuté par le processeur (292), le programme informatique amène en outre la console (290) à générer (S880) un affichage des images de la sonde d'imagerie par ultrasons (210) synchronisé avec les emplacements du premier capteur passif à ultrasons (S1).

19. Support de stockage tangible non transitoire lisible par ordinateur (291) selon la revendication 16, dans lequel le second signal comprend une forme d'onde prédéfinie et, lorsqu'il est exécuté par le processeur (292), le programme informatique amène en outre la console (290) à détecter la forme d'onde prédéfinie dans le premier signal combiné.

20. Support de stockage tangible non transitoire lisible par ordinateur (291) de la revendication 16, dans lequel le second signal comprend une première forme d'onde prédéfinie et une seconde forme d'onde prédéfinie, lorsqu'il est exécuté par le processeur (292), le programme informatique amène en outre la console (190/290/390) à détecter la première forme d'onde prédéfinie et la seconde forme d'onde prédéfinie dans le premier signal combiné, la première forme d'onde prédéfinie correspond à un déclencheur de trame et la seconde forme d'onde prédéfinie correspond à un déclencheur de ligne.
